# EUROPEAN PATENT APPLICATION

(11) **EP 4 524 577 A1**
(43) Date of publication of application: **19.03.2025**
(21) Application number: 24200224.4
(22) Date of filing: 13.09.2024
(51) Int. Cl.: G01N 35/00, G01N 15/14, G01N 15/10, G01N 15/01

(54) **ANALYZER**

(30) Priority: 15.09.2023 JP 2023003396 U; 15.09.2023 JP 2023150301
(71) Applicant: SYSMEX CORPORATION, Kobe-shi Hyogo 651-0073 (JP)
(72) Inventor: IKUTA, Junya, Kobe-shi, Hyogo, 651-0073 (JP); NAKANISHI, Noriyuki, Kobe-shi, Hyogo, 651-0073 (JP); MISAWA, Kota, Kobe-shi, Hyogo, 651-0073 (JP); MIZUHASHI, Toru, Kobe-shi, Hyogo, 651-0073 (JP)
(74) Representative: Hoffmann Eitle

(57) **Abstract**

Disclosed is an analyzer configured to analyze a specimen, including: first and second sample preparation parts configured to prepare a first measurement sample from the specimen by using a first reagent; a third sample preparation part configured to prepare a second measurement sample from the specimen by using a second reagent; an optical measurement part configured to measure the first measurement sample to obtain an optical signal corresponding to white blood cells; an electric measurement part configured to measure the second measurement sample to obtain an electric signal corresponding to red blood cells; and a controller programmed to analyze the specimen, based on the optical signal and the electric signal. The controller is programmed to, according to a plurality of measurement orders, control sample preparation in the first and second sample preparation parts and sample measurement by the optical measurement part such that at least at least a part of a first period overlaps with at least a part of a second period, the first period including a preparation of the first measurement sample by the first sample preparation part and a measurement thereof by the optical measurement part, and the second period including a preparation of the first measurement sample by the second sample preparation part and a measurement thereof by the optical measurement part.

## Description

### CROSS REFERENCE TO RELATED APPLICATIONS

This application claims priority from prior Japanese Patent Application No. 2023-150301, filed on September 15, 2023, entitled "ANALYZER", and prior Japanese Utility Model Application No. 2023-003396, filed on September 15, 2023, entitled "ANALYZER", the entire contents of which are incorporated herein by reference.

### FIELD OF THE INVENTION

The present invention relates to an analyzer for analyzing a specimen.

### BACKGROUND OF THE INVENTION

Gabriella Lakos, "Calibration and QC Guidance for the Abbott Alinity hq Hematology Analyzer", (U.S.), Abbott (Non-Patent Literature 1) discloses an analyzer for measuring and classifying cells in a blood specimen. This analyzer includes two systems of mechanisms for mixing specimens and reagents to prepare measurement samples. Each of the two systems of mechanisms includes a container for preparing a measurement sample for red blood cell (RBC) measurement and a container for preparing a measurement sample for white blood cell (WBC) measurement. Both the RBC measurement sample and the WBC measurement sample are measured by a flow cytometer. Since this analyzer includes the two systems of mechanisms for preparing measurement samples, sample preparation can be performed by one mechanism while sample preparation and measurement are being performed in the other mechanism, whereby throughput of the analyzer is improved.

Yutaka Nagai, "Studies on human nucleated cell structure analysis based on light scattering measurements", October 2006 (Non-Patent Literature 2) discloses a result of comparison between accuracies of optical RBC measurement and electric RBC measurement. Non-Patent Literature 2 indicates that the accuracy of RBC measurement is higher in the electric measurement than in the optical measurement.

The analyzer of Non-Patent Literature 1 includes the two systems of mechanisms for sample preparation to improve the throughput. However, since the flow cytometer based on optical measurement is used for RBC measurement, the accuracy of RBC measurement is inferior to that of the electric measurement.

In view of the above problem, an object of the present invention is to provide an analyzer capable of improving throughput of specimen analysis and accuracy of RBC measurement.

### SUMMARY OF THE INVENTION

An analyzer (1) of the present invention is an analyzer for analyzing a specimen. The analyzer (1) of the present invention includes: first and second sample preparation parts (C21, C22) configured to prepare a first measurement sample from the specimen by using a first reagent; a third sample preparation part (C11) configured to prepare a second measurement sample from the specimen by using a second reagent; an optical measurement part (381) configured to measure the first measurement sample to obtain an optical signal corresponding to white blood cells; an electric measurement part (382) configured to measure the second measurement sample to obtain an electric signal corresponding to red blood cells; and a controller (852) programmed to analyze the specimen, based on the optical signal and the electric signal. The controller (852) is further programmed to, according to a plurality of measurement orders, control sample preparation in the first and second sample preparation parts (C21, C22) and sample measurement by the optical measurement part (381) such that at least a part of a first period overlaps with a second period, the first period including a preparation of the first measurement sample by the first sample preparation part (C21) and measurement thereof by the optical measurement part (381) and the second period including a preparation of the first measurement sample by the second sample preparation part (C22) and measurement thereof by the optical measurement part (381).

According to the analyzer of the present invention, since a plurality of sample preparation parts for preparing measurement samples to be measured by the optical measurement part are provided, at least parts of the periods for sample preparation and measurement can be overlapped with each other. Thus, throughput of specimen analysis can be improved. In addition, since the electric measurement part is used for measuring red blood cells, accuracy of RBC measurement can be improved.

According to the present invention, throughput of specimen analysis and measurement accuracy for red blood cells can be improved.

### BRIEF DESCRIPTION OF THE DRAWINGS

FIG. 1 is a perspective view showing a configuration of an analyzer according to an embodiment;
FIG. 2 is a perspective view showing configurations of a specimen rack and specimen containers according to the embodiment;
FIG. 3 is a plan view schematically showing a configuration of a transport unit according to the embodiment;
FIG. 4 is a plan view schematically showing a configuration of a measurement unit inside a housing, according to the embodiment;
FIG. 5 is a block diagram showing a configuration in which measurement samples prepared in reaction chambers provided in the measurement unit are supplied to an optical measurement part, an electric measurement part, and an HGB measurement part, according to the embodiment;
FIG. 6 is a schematic diagram illustrating the order in which the specimen suctioned by a suction tube is dispensed to the reaction chambers, according to the embodiment;
FIG. 7 is a diagram schematically showing a configuration of a liquid transfer unit for suctioning and discharging the specimen through the suction tube, according to the embodiment;
FIG. 8 is a diagram schematically showing a configuration of a fluid circuit connected to a WDF reaction chamber, a WNR reaction chamber, an RET/PLT-F reaction chamber, and an optical measurement part, according to the embodiment;
FIG. 9 is a diagram schematically showing a configuration of a fluid circuit connected to an RBC/PLT reaction chamber, an HGB reaction chamber, an electric measurement part, and an HGB measurement part, according to the embodiment;
FIG. 10 is a perspective view showing configurations of a WDF reaction chamber, a WNR reaction chamber, and an RET/PLT-F reaction chamber, according to the embodiment;
FIG. 11 is an exploded perspective view showing a configuration of a reaction chamber heating unit according to the embodiment;
FIG. 12 is a perspective view showing the configuration of the reaction chamber heating unit that has been assembled, according to the embodiment;
FIG. 13 is a perspective view showing a configuration of a frame portion included in a reagent heating unit according to the embodiment;
FIG. 14 is an exploded perspective view showing a configuration of the reagent heating unit according to the embodiment;
FIG. 15 is a perspective view showing a configuration of a reagent heating unit that has been assembled, according to the embodiment;
FIG. 16 is an exploded perspective view showing a configuration of a heating unit according to the embodiment;
FIG. 17 is a perspective view showing the configuration of the heating unit that has been assembled, according to the embodiment;
FIG. 18 is a diagram showing a configuration of a flow cell of the optical measurement part, a configuration of a flow cell of the electric measurement part, and a configuration of the HGB measurement part, according to the embodiment;
FIG. 19 is a block diagram showing a functional configuration of the measurement unit according to the embodiment;
FIG. 20 is a block diagram showing functional configurations of the transport unit and an analysis unit according to the embodiment;
FIG. 21 is a schematic diagram showing a configuration of a screen displayed on a display part of the analysis unit, according to the embodiment;
FIG. 22 is a schematic diagram showing a configuration of a screen displayed on a display part of an analysis unit according to the embodiment;
FIG. 23 is a time chart in a case where a measurement order of CBC+DIFF is set for each of two specimens to be consecutively measured, according to the embodiment;
FIG. 24 is a time chart in a case where a measurement order of CBC+DIFF (without WNR) is set for each of two specimens to be consecutively measured, according to the embodiment;
FIG. 25 is a time chart in a case where a measurement order of CBC+DIFF+RET is set for each of two specimens to be consecutively measured, according to the embodiment;
FIG. 26 is a flowchart showing an operation of the analyzer regarding a specimen contained in one specimen container, according to the embodiment;
FIG. 27 is a flowchart showing a process of the analysis unit for specifying an operation sequence, according to the embodiment;
FIG. 28 is a time chart showing an example in which operation sequences of two specimens to be consecutively measured are started at start timings, according to the embodiment; and
FIG. 29 is a time chart showing an example in which an operation sequence of a specimen shown in FIG. 28 is not started at a start timing, according to the embodiment.

### DETAILED DESCRIPTION

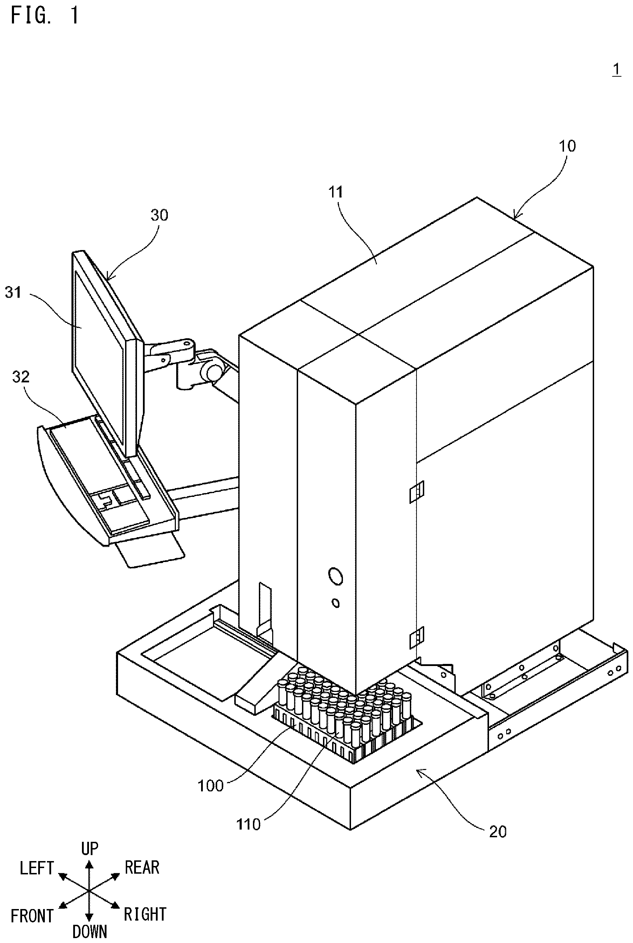

FIG. 1 is a perspective view showing the configuration of an analyzer 1. In FIG. 1, up, down, left, right, front, and rear directions are shown.

The analyzer 1 is a blood cell counter that measures white blood cells, red blood cells, platelets, etc., contained in a specimen, and counts each blood cell. The specimen is whole blood collected from a subject. The analyzer 1 includes a measurement unit 10, a transport unit 20, and an analysis unit 30.

The measurement unit 10 measures blood cells contained in the specimen. Various components for measuring the specimen are housed in a housing 11 of the measurement unit 10. The transport unit 20 is disposed on the front side of the measurement unit 10, and transports a specimen rack 100 holding a plurality of specimen containers 110.

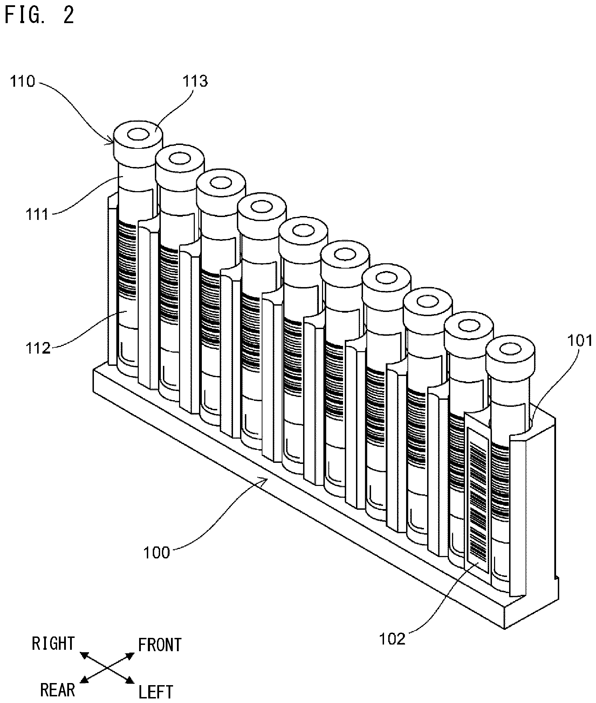

FIG. 2 is a perspective view showing the configurations of the specimen rack 100 and the specimen containers 110.

The specimen rack 100 has ten holes 101 in which the specimen containers 110 can be held, and a bar code label 102. The bar code label 102 is adhered to the rear-side surface of the specimen rack 100. On the bar code label 102, a bar code indicating a rack ID that allows individual identification of the specimen rack 100 is printed.

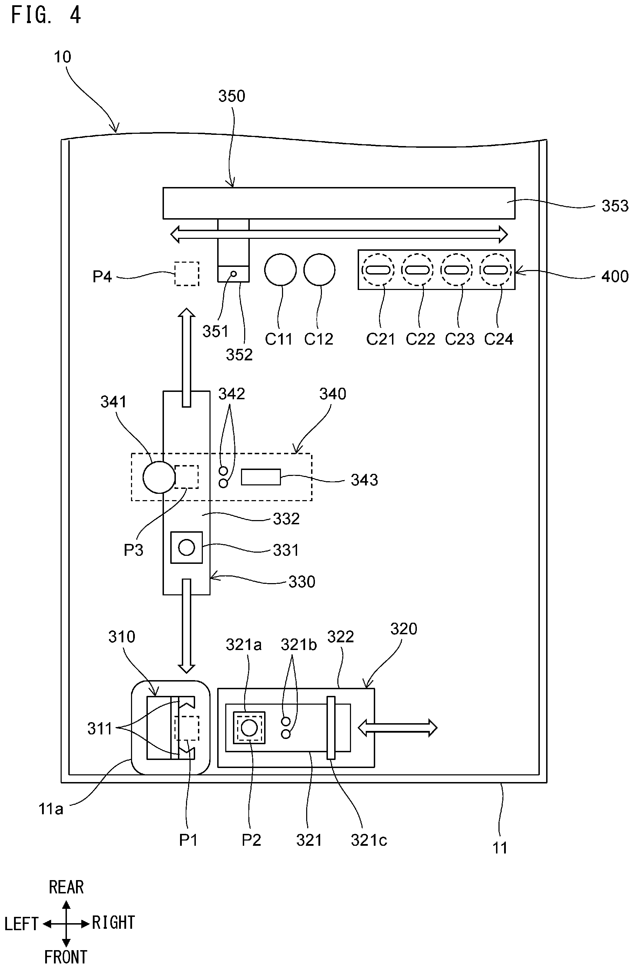

The specimen container 110 includes a trunk portion 111, a bar code label 112, and a lid portion 113. The trunk portion 111 is a tubular container having an opened upper end, and stores a specimen therein. The bar code label 112 is adhered to the side surface of the trunk portion 111. On the bar code label 112, a bar code indicating a specimen ID that allows individual identification of the contained specimen is printed. The lid portion 113 is disposed on the upper end of the trunk portion 111 to seal the inside of the trunk portion 111. The lid portion 113 is provided with an elastic material through which a suction tube 351 (see FIG. 4) provided in the measurement unit 10 can vertically penetrate.

Referring back to FIG. 1, the transport unit 20 transports the specimen rack 100, and supplies the specimen containers 110 held in the specimen rack 100 to the measurement unit 2. The measurement unit 10 takes out a specimen container 110 from the specimen rack 100, and transfers the taken-out specimen container 110 into the housing 11. The measurement unit 10 measures the specimen in the specimen container 110, and returns the specimen container 110 for which the measurement has ended, to the specimen rack 100.

The analysis unit 30 performs an analysis including counting of blood cells, etc., based on the measurement result obtained by the measurement unit 10, and generates an analysis result. The analysis result includes, for example, a numerical value result, a graph, and a chart that are based on the analysis, flag information provided to the specimen, and the like. The analysis unit 30 includes a display part 31 and an input part 32. The display part 31 displays the analysis result, etc., and the input part 32 receives an input from an operator.

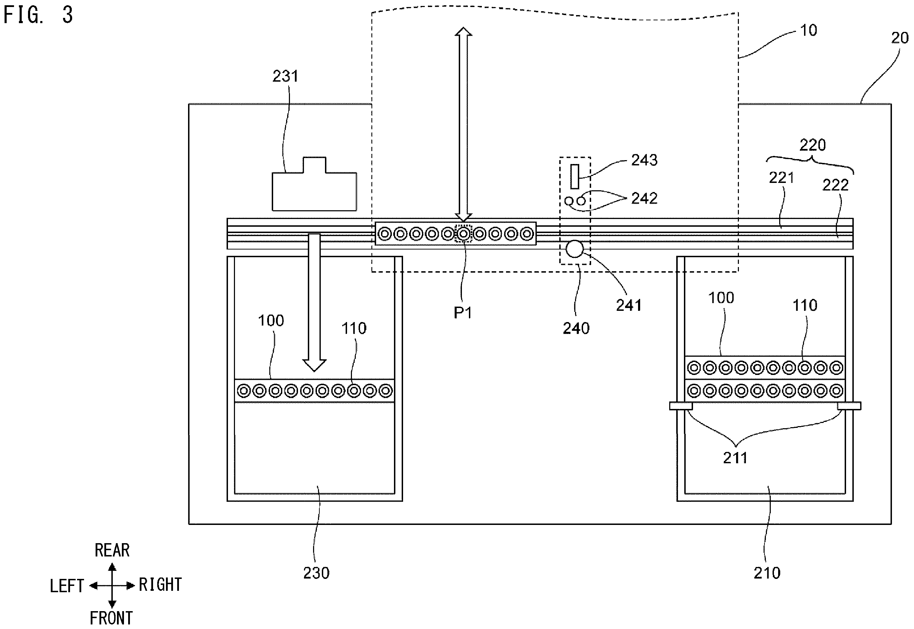

FIG. 3 is a plan view schematically showing the configuration of the transport unit 20.

The transport unit 20 includes a placement region 210, a transport path 220, a collection region 230, and a reading mechanism 240.

The operator sets a specimen container 110 containing specimens to be analyzed into holes 101 of the specimen rack 100, and places the specimen rack 100 in which the specimen containers 110 are set, on the placement region 210. The placement region 210 is provided with a transport mechanism 211 that pushes out the specimen rack 100 rearward. The transport mechanism 211 includes a pair of members for pushing the front surface of the specimen rack 100. The specimen rack 100 pushed rearward by the transport mechanism 211 is positioned at the right end of the transport path 220.

The transport path 220 includes two conveyor belts 221, 222 that independently move in the left-right direction. The conveyor belts 221, 222 transfer the specimen rack 100 positioned at the right end of the transport path 220 to a predetermined position on the transport path 220. The reading mechanism 240 includes a roller 241, a pair of rollers 242, a bar code reader 243, a mechanism for rotating the roller 241, and a mechanism for moving the pair of rollers 242 in the front-rear direction. The reading mechanism 240 reads the rack ID and the specimen IDs from the specimen rack 100 and the specimen containers 110 on the transport path 220. The configuration of the reading mechanism 240 is identical to that of a reading mechanism 340 (see FIG. 4) described below.

The transport path 220 is provided with a take-out position P1 for the measurement unit 10 to take out a specimen container 110. When a specimen container 110 is positioned at the take-out position P1, the measurement unit 10 takes out this specimen container 110 from the specimen rack 100, takes the specimen container 110 into the measurement unit 10, suctions the specimen in the specimen container 110, mixes the suctioned specimen with a reagent to prepare a measurement sample, and measures the measurement sample to obtain a measurement result. Thereafter, the analysis unit 30 analyzes the specimen, based on the measurement result. When suction of the taken specimen container 110 has ended, the measurement unit 10 returns the specimen container 110 back to the original hole 101 of the specimen rack 100. When all the specimen containers 110 held in the specimen rack 100 have been subjected to required analyses, this specimen rack 100 is transported to the left end of the transport path 220.

The collection region 230 is provided with a transport mechanism 231 that pushes out the specimen rack 100 frontward. The transport mechanism 231 has a member for pushing the specimen rack 100 rearward. The specimen rack 100 positioned at the left end of the transport path 220 is pushed out frontward by the transport mechanism 231, and is collected into the collection region 230. The operator takes out the specimen rack 100 collected in the collection region 230. Thus, transport of the specimen rack 100 and analyses of the specimens in the specimen containers 110 held in the specimen rack 100 are ended.

FIG. 4 is a plan view schematically showing the configuration of the measurement unit 10 inside the housing 11.

The measurement unit 10 includes a gripping mechanism 310, an agitation mechanism 320, a container transfer mechanism 330, a reading mechanism 340, a dispensing mechanism 350, an RBC/PLT reaction chamber C11, an HGB reaction chamber C12, and a reaction chamber heating unit 400.

The gripping mechanism 310 includes a pair of gripping pieces 311 for gripping a specimen container 110 in the front-rear direction, a mechanism that causes the pair of gripping pieces 311 to move close to and away from each other, and a mechanism that causes the pair of gripping pieces 311 to move in the up-down direction. The gripping mechanism 310 is disposed above the take-out position P1 on the transport path 220 (see FIG. 3). At the lower surface of the housing 11, an opening 11a is formed to make the pair of gripping pieces 311 vertically movable with respect to the lower surface of the housing 11.

The gripping mechanism 310 grips the specimen container 110 positioned at the take-out position P1 by using the pair of gripping pieces 311, and moves the pair of gripping pieces 311 upward to take out the specimen container 110 from the specimen rack 100 and position the specimen container 110 above the take-out position P1.

The agitation mechanism 320 includes a holding part 321, a slide part 322, and a mechanism that drives the holding part 321 and the slide part 322. The holding part 321 includes a holding member 321a in which a hole capable of holding a specimen container 110 is formed, a roller 321b movable in the left-right direction, and a rotation shaft 321c extending in the front-rear direction. The slide part 322 supports the lower portion of the holding part 321, and is configured to be movable in the left-right direction. The holding part 321 is configured to be rotatable around the rotation shaft 321c.

When the gripping mechanism 310 takes out the specimen container 110 at the take-out position P1 from the specimen rack 100 and transfers the specimen container 110 upward, the agitation mechanism 320 transfers the slide part 322 leftward so that the holding member 321a is positioned below the specimen container 110. In this state, the gripping mechanism 310 moves the pair of gripping pieces 311 downward so that the specimen container 110 being gripped by the gripping pieces 311 is set in the holding member 321a. Subsequently, the agitation mechanism 320 transfers the slide part 322 rightward so that the specimen container 110 being held in the holding member 321a is positioned at an agitation position P2. Then, the agitation mechanism 320 moves the roller 321b leftward to keep the specimen container 110 inside the holding member 321a. In this state, the agitation mechanism 320 rotates the holding part 321 around the rotation shaft 321c to agitate the specimen in the specimen container 110 held in the holding member 321a.

The container transfer mechanism 330 includes a holding member 331 in which a hole capable of holding a specimen container 110 is formed, a plate member 332 that supports the holding member 331 and extends in the front-rear direction, and a mechanism that moves the plate member 332 in the front-rear direction.

When agitation of the specimen container 110 has ended, the agitation mechanism 320 again positions the specimen container 110 held in the holding member 321a, above the take-out position P1, and the gripping mechanism 310 takes out the specimen container 110 upward from the holding member 321a. In this state, the agitation mechanism 320 retracts the holding part 321 and the slide part 322 rightward, and the container transfer mechanism 330 positions the holding member 331 under the specimen container 110. Then, the gripping mechanism 310 moves the pair of gripping pieces 311 downward to set the specimen container 110 in the holding member 331. Thereafter, the container transfer mechanism 330 positions the specimen container 110 held in the holding member 331, to a reading position P3 of the reading mechanism 340.

The reading mechanism 340 includes a roller 341, a pair of rollers 342, a bar code reader 343, a mechanism that rotates the roller 341, and a mechanism that moves the pair of rollers 342 in the left-right direction.

When the specimen container 110 is positioned at the reading position P3, the reading mechanism 340 moves the pair of rollers 342 leftward to sandwich the specimen container 110 with the roller 341 and the pair of rollers 342. Subsequently, the reading mechanism 340 reads the specimen ID from the specimen container 110 with the bar code reader 343 while rotating the specimen container 110 with rotation of the roller 341. The reading mechanism 240 provided on the transport path 220 has the same configuration as the reading mechanism 340. Thereafter, the container transfer mechanism 330 positions the specimen container 110 held in the holding member 331 at a suction position P4 of the dispensing mechanism 350.

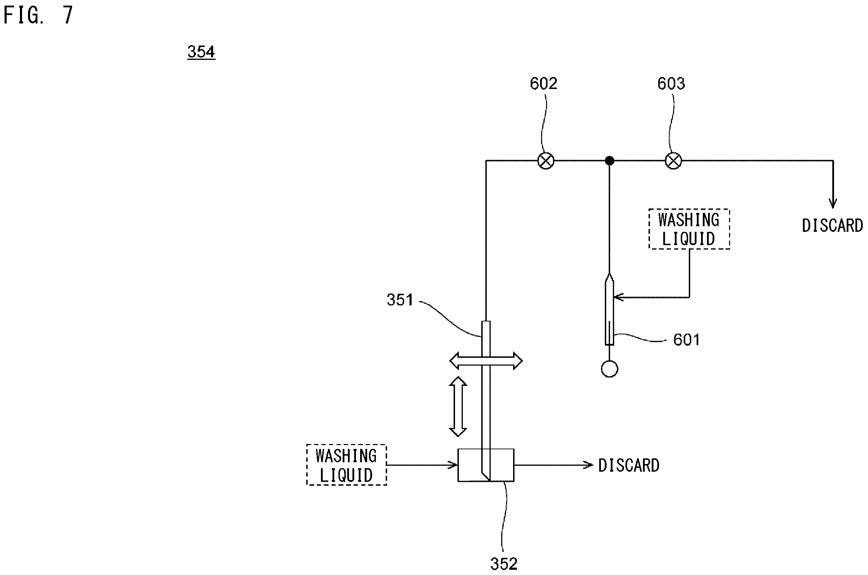

The dispensing mechanism 350 includes a suction tube 351 that extends in the up-down direction and has high rigidity, a washer 352 for washing the suction tube 351, a transfer part 353 that transfers the suction tube 351 in the up-down direction and the left-right direction, and a liquid transfer part 354 (see FIG. 7) that suctions and discharges the specimen through the suction tube 351. The suction tube 351 is used for suctioning the specimen from the specimen container 110 at the suction position P4, and dispensing the suctioned specimen.

When the specimen container 110 is positioned at the suction position P4, the dispensing mechanism 350 drives the transfer part 353 to move the suction tube 351 downward so that the lower end of the suction tube 351 penetrates through the lid portion 113 (see FIG. 2) of the specimen container 110. At this time, the lower end of the suction tube 351 is positioned a predetermined distance below the liquid surface in the specimen container 110. In this state, the dispensing mechanism 350 suctions the specimen in the specimen container 110 through the suction tube 351.

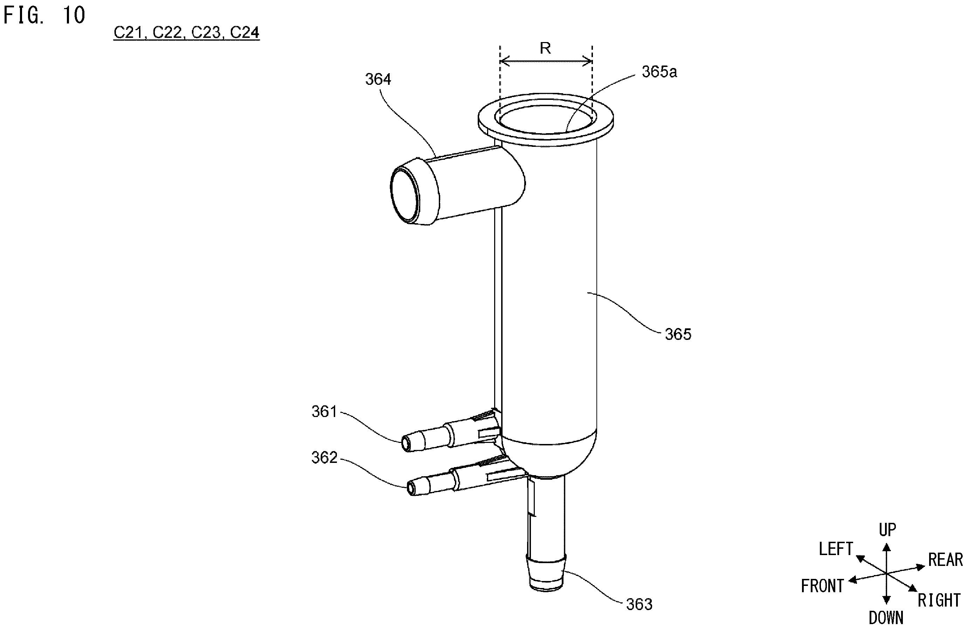

The reaction chamber heating unit 400 includes WDF reaction chambers C21, C22, a WNR reaction chamber C23, and an RET/PLT-F reaction chamber C24. In the reaction chamber heating unit 400, the WDF reaction chambers C21, C22 are arranged adjacent to each other. The RBC/PLT reaction chamber C11 and the HGB reaction chamber C12 are disposed between the suction position P4 and the WDF reaction chamber C21. The reaction chambers C11, C12, C21, C22, C23, C24 are arranged closer to the suction position P4 in this order, and are arranged side by side in a straight line in the left-right direction. The detailed configuration of the reaction chamber heating unit 400 will be described later with reference to FIGS. 10 to 12.

The dispensing mechanism 350 dispenses the specimen suctioned through the suction tube 351 at the suction position P4, into at least one reaction chamber among the reaction chambers C11, C12, C21 to C24, based on a measurement order set on the specimen. In each of the reaction chambers C11, C12, C21 to C24, the specimen is mixed with a predetermined reagent, whereby a measurement sample is prepared.

When suction of the specimen from the specimen container 110 has ended, the container transfer mechanism 330 positions the specimen container 110 held in the holding member 331, above the take-out position P1, and the gripping mechanism 310 grips and lifts the specimen container 110 upward from the holding member 331. In this state, the container transfer mechanism 330 retracts the holding member 331 rearward, and the gripping mechanism 310 returns the gripped specimen container 110 to the original hole 101 of the specimen rack 100.

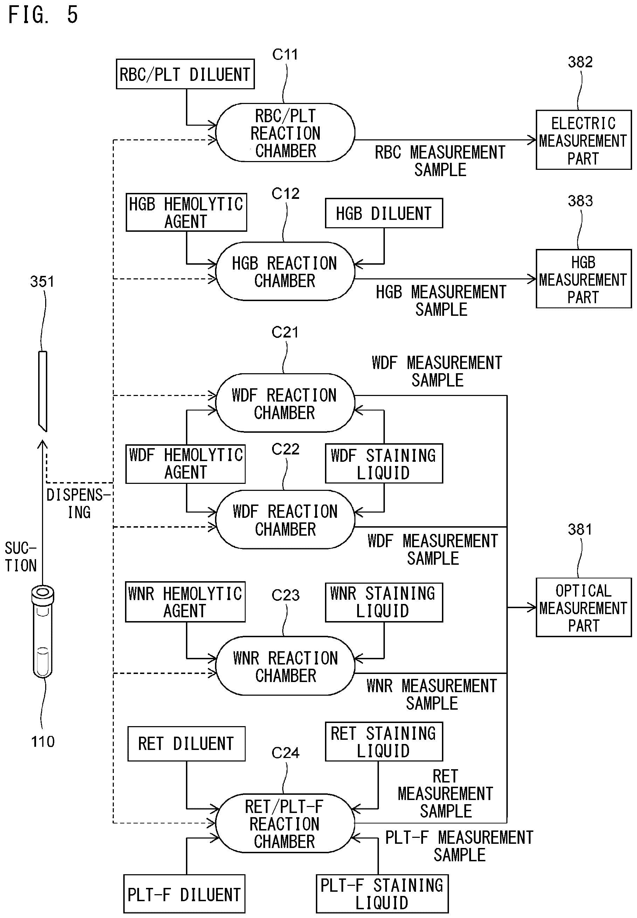

FIG. 5 is a block diagram showing a configuration in which the measurement samples prepared in the respective reaction chambers C11, C12, C21 to C24 provided in the measurement unit 10 are supplied to an optical measurement part 381, an electric measurement part 382, and an HGB measurement part 383.

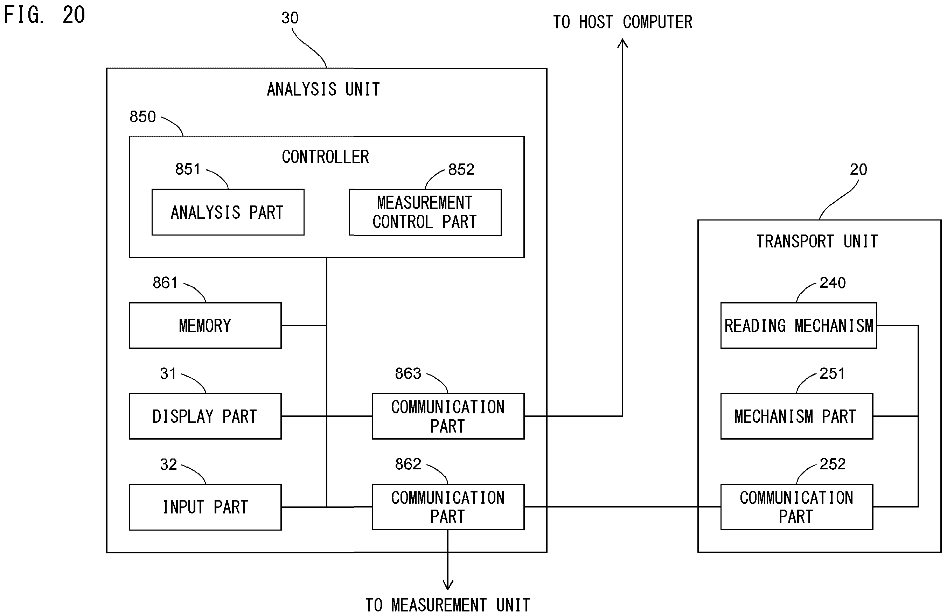

The operations excluding analysis which are described with reference to FIG. 5 are performed by a measurement control part 852 (see FIG. 20), of the analysis unit 30, which controls the dispensing mechanism 350, preparation of the measurement samples in the reaction chambers C11, C12, C21 to C24, and measurement by the measurement parts 381 to 383.

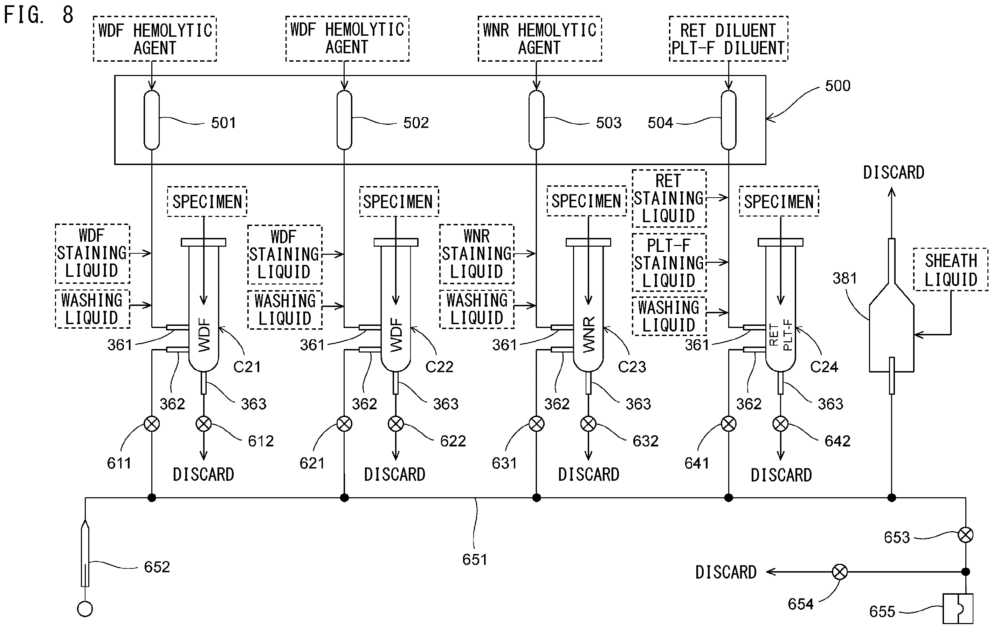

The specimen suctioned from the specimen container 110 by the suction tube 351 is dispensed into the reaction chambers C11, C12, C21 to C24 as appropriate, based on the measurement order set on the specimen. The reaction chambers C11, C12, C21 to C24 are containers having opened upper ends. The configurations of the reaction chambers C11, C12, C21 to C24 will be described later with reference to FIGS. 8 and 10.

In the RBC/PLT reaction chamber C11, the specimen is mixed with an RBC/PLT diluent to prepare an RBC/PLT measurement sample. The RBC/PLT diluent is, for example, Cellpack (registered trademark) DCL. The RBC/PLT measurement sample prepared in the RBC/PLT reaction chamber C11 is measured by the electric measurement part 382. The electric measurement part 382 obtains electric signals corresponding to red blood cells and platelets. An analysis part 851 (see FIG. 20) of the analysis unit 30 analyzes the measurement result of the RBC/PLT measurement sample, and obtains the number of red blood cells, the number of platelets, and the like.

In the HGB reaction chamber C12, the specimen is mixed with an HGB hemolytic agent and an HGB diluent to prepare an HGB measurement sample. The HGB hemolytic agent is, for example, Sulfolyser (registered trademark), and the HGB diluent is, for example, Cellpack (registered trademark) DCL. The HGB measurement sample prepared in the HGB reaction chamber C12 is measured by the HGB measurement part 383. The HGB measurement part 383 obtains a signal corresponding to hemoglobin concentration. The analysis part 851 of the analysis unit 30 analyzes the measurement result of the HGB measurement sample, and obtains the hemoglobin concentration, and the like.

In the WDF reaction chamber C21, the specimen is mixed with a WDF hemolytic agent and a WDF staining liquid to prepare a WDF measurement sample. The WDF hemolytic agent is, for example, Lysercell (registered trademark) WDF II, and the WDF staining liquid is, for example, Fluorocell (registered trademark) WDF. The WDF measurement sample prepared in the WDF reaction chamber C21 is measured by the optical measurement part 381. The optical measurement part 381 obtains an optical signal corresponding to white blood cells. The analysis part 851 of the analysis unit 30 analyzes the measurement result of the WDF measurement sample prepared in the WDF reaction chamber C21 and the measurement result of a WNR measurement sample, and performs, for example, classification of white blood cells (into five types including neutrophils, lymphocytes, monocytes, eosinophils, and basophils, for example).

In the WDF reaction chamber C22, as in the WDF reaction chamber C21, the specimen is mixed with a WDF hemolytic agent and a WDF staining liquid to prepare a WDF measurement sample. The WDF measurement sample prepared in the WDF reaction chamber C22 is measured by the optical measurement part 381. The optical measurement part 381 obtains an optical signal corresponding to white blood cells. The analysis part 851 of the analysis unit 30 analyzes the measurement result of the WDF measurement sample prepared in the WDF reaction chamber C22, and the measurement result of the WNR measurement sample, and performs, for example, classification of white blood cells (into five types including neutrophils, lymphocytes, monocytes, eosinophils, and basophils, for example).

Here, in the WDF reaction chambers C21, C22, the WDF measurement samples are prepared under the same preparation conditions. Specifically, to each of the WDF reaction chambers C21, C22, a WDF hemolytic agent and a WDF staining liquid are supplied as reagents of the same type. To each of the WDF reaction chambers C21, C22, the WDF hemolytic agent is supplied from a common reagent container and the WDF staining liquid is supplied from a common reagent container. The amount of specimen to be dispensed, the amount of reagent to be supplied, the time required for preparing the WDF measurement sample, the amount of prepared WDF measurement sample, and the reaction temperature in preparation are common between the WDF reaction chambers C21, C22.

In the WNR reaction chamber C23, the specimen is mixed with a WNR hemolytic agent and a WNR staining liquid to prepare a WNR measurement sample. The WNR hemolytic agent is, for example, Lysercell (registered trademark) WNR, and the WNR staining liquid is, for example, Fluorocell (registered trademark) WNR. The WNR measurement sample prepared in the WNR reaction chamber C23 is measured by the optical measurement part 381. The optical measurement part 381 obtains an optical signal corresponding to white blood cells and nucleated red blood cells. The analysis part 851 of the analysis unit 30 analyzes the measurement result of the WNR measurement sample, and obtains the number of white blood cells, the number of nucleated red blood cells, etc.

In the RET/PLT-F reaction chamber C24, the specimen is mixed with an RET diluent and an RET staining liquid to prepare an RET measurement sample. The RET diluent is, for example, Cellpack (registered trademark) DFL, and the RET staining liquid is, for example, Fluorocell (registered trademark) RET. The RET measurement sample prepared in the RET/PLT-F reaction chamber C24 is measured by the optical measurement part 381. The optical measurement part 381 obtains an optical signal corresponding to reticulocytes. The analysis part 851 of the analysis unit 30 analyzes the measurement result of the RET measurement sample, and performs, for example, classification of reticulocytes.

In the RET/PLT-F reaction chamber C24, the specimen is mixed with a PLT-F diluent and a PLT-F staining liquid to prepare a PLT-F measurement sample. The PLT-F diluent is, for example, Cellpack (registered trademark) DFL, and the PLT-F staining liquid is, for example Fluorocell (registered trademark) PLT. The PLT-F measurement sample prepared in the RET/PLT-F reaction chamber C24 is measured by the optical measurement part 381. The optical measurement part 381 obtains an optical signal corresponding to platelets. The analysis part 851 of the analysis unit 30 analyzes the measurement result of the PLT-F measurement sample, and obtains the number of platelets, etc.

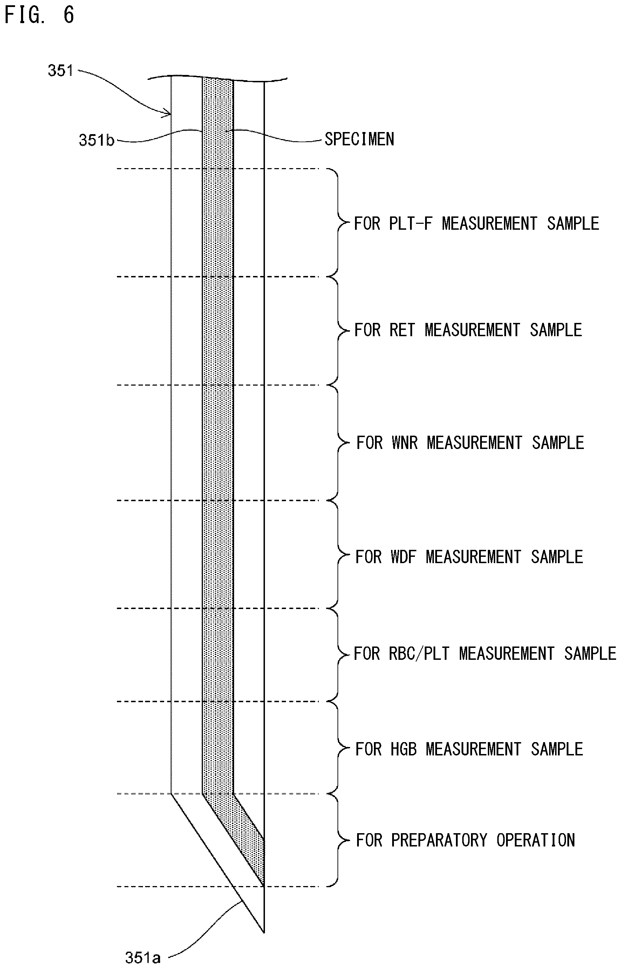

FIG. 6 is a schematic diagram illustrating the order in which the specimen suctioned by the suction tube 351 is dispensed into the respective reaction chambers.

A lower end portion 351a of the suction tube 351 has a shape obtained by cutting out a lower end of a cylindrical shape by a plane inclined with respect to a horizontal plane. A flow path 351b through which the specimen passes is formed inside the suction tube 351. The flow path 351b is formed along the up-down direction in which the suction tube 351 extends, and is opened to the outside from the side surface of the suction tube 351 at the lower end portion 351a.

The amount of specimen to be suctioned from the specimen container 110 by the suction tube 351 is constant regardless of the measurement order set on the specimen. When a certain amount of specimen has been suctioned from the specimen container 110 by the suction tube 351, the flow path 351b is filled with the specimen as shown in FIG. 6. The specimen in the flow path 351b is discharged from the lower end portion 351a side into predetermined reaction chambers in order. This dispensing operation is continuously performed in response to one suction of specimen from the specimen container 110.

Specifically, of the specimen in the flow path 351b, a predetermined amount of specimen positioned at the lowest position is discharged to the RBC/PLT reaction chamber C11 as a preparatory operation for dispensing by the suction tube 351. The specimen discharged to the RBC/PLT reaction chamber C11 through the preparatory operation is discarded through a flow path for discarding described later. Subsequently, a predetermined amount of specimen positioned above the specimen for the preparatory operation in the flow path 351b is discharged to the HGB reaction chamber C12 as a specimen for preparing an HGB measurement sample. Subsequently, a predetermined amount of specimen positioned above the specimen for the HGB measurement sample in the flow path 351b is discharged to the RBC/PLT reaction chamber C1 1 as a specimen for preparing an RBC/PLT measurement sample. Subsequently, a predetermined amount of specimen positioned above the specimen for an RBC/PLT measurement sample in the flow path 351b is discharged to the WDF reaction chamber C21 or C22 as a sample for preparing a WDF measurement sample. Subsequently, a predetermined amount of specimen positioned above the specimen for the WDF measurement sample in the flow path 351b is discharged to the WNR reaction chamber C23 as a specimen for preparing a WNR measurement sample. Subsequently, a predetermined amount of specimen positioned above the specimen for the WNR measurement sample in the flow path 351b is discharged to the RET/PLT-F reaction chamber C24 as a specimen for preparing an RET measurement sample. Subsequently, a predetermined amount of specimen positioned above the specimen for the RET measurement sample in the flow path 351b is discharged to the RET/PLT-F reaction chamber C24 as a specimen for preparing a PLT-F measurement sample.

In the dispensing operation as described above, the amount of the specimen to be discharged to each reaction chamber is determined to be a predetermined amount corresponding to the preparatory operation and the preparation of each measurement sample. In addition, a specimen corresponding to a measurement sample that does not need to be prepared is discarded without being used for preparation of the measurement sample. For example, if preparation of an RET measurement sample is not needed, the specimen for the RET measurement sample is discharged from an opening of the suction tube 351 with a tip of the suction tube 351 being positioned at the height of the washer 352, and is collected by the washer 352. Discarding of the unnecessary specimen in the suction tube 351 may not necessarily use the washer 352. The unnecessary specimen may be discharged into an empty reaction chamber to be discarded from the reaction chamber.

The reason why the order of dispensing into the respective reaction chambers is determined as shown in FIG. 6 is as follows. That is, when the specimen is suctioned into the suction tube 351, a concentration gradient occurs in the up-down direction in the flow path 351b of the suction tube 351. Since the amount of specimen to be suctioned by the suction tube 351 is constant, the concentration of specimen to be dispensed into each reaction chamber can be set to be substantially constant for each measurement, unless the order of dispensing into the reaction chambers is changed. Thus, variations in measurement results due to concentration gradients can be inhibited.

FIG. 7 schematically shows the configuration of the liquid transfer part 354 for suctioning and discharging a specimen through the suction tube 351.

An upper end of the suction tube 351 is connected to a syringe pump 601 via a flow path, and a valve 602 is disposed in the flow path. The syringe pump 601 includes a piston and a motor, and applies a predetermined pressure to the flow path to suction a predetermined amount of specimen from the suction tube 351, and dispense only a predetermined amount of the specimen suctioned into the suction tube 351. When dispensing of the specimen has ended, the syringe pump 601 draws the specimen remaining in the suction tube 351 and discards the specimen through the valve 603.

In washing the flow path 351b of the suction tube 351 (see FIG. 6), the lower end portion 351a of the suction tube 351 (see FIG. 6) is positioned inside the washer 352. In this state, the syringe pump 601 transfers, to the suction tube 351, a washing liquid supplied to the syringe pump 601. The washing liquid transferred to the suction tube 351 is discharged from the opening of the lower end portion 351a, and is discarded through the washer 352. In washing the outer side surface of the suction tube 351, the suction tube 351 is moved in the up-down direction with respect to the washer 352. At this time, the washing liquid supplied to the washer 352 hits the outer side surface of the suction tube 351, and is discarded through the washer 352. Thus, the inside and the outside of the suction tube 351 are washed.

FIG. 8 schematically shows the configuration of a fluid circuit connected to the WDF reaction chambers C21, C22, the WNR reaction chamber C23, the RET/PLT-F reaction chamber C24, and the optical measurement part 381.

The reaction chambers C21 to C24 have the same configuration. Each of the reaction chambers C21 to C24 includes an inlet 361 to which a reagent and a washing liquid are supplied, an outlet 362 from which a measurement sample prepared in the reaction chamber is discharged, and a disposal port 363 from which the liquid in the reaction chamber is discharged.

The WDF hemolytic agent, the WDF staining liquid, and the washing liquid are supplied to the WDF reaction chamber C21 through the inlet 361. The WDF hemolytic agent, the WDF staining liquid, and the washing liquid are also supplied to the WDF reaction chamber C22 through the inlet 361, like the WDF reaction chamber C21. The WNR hemolytic agent, the WNR staining liquid, and the washing liquid are supplied to the WNR reaction chamber C23 through the inlet 361. The RET diluent, the PLT-F diluent, the RET staining liquid, the PLT-F staining liquid, and the washing liquid are supplied to the RET/PLT-F reaction chamber C24.

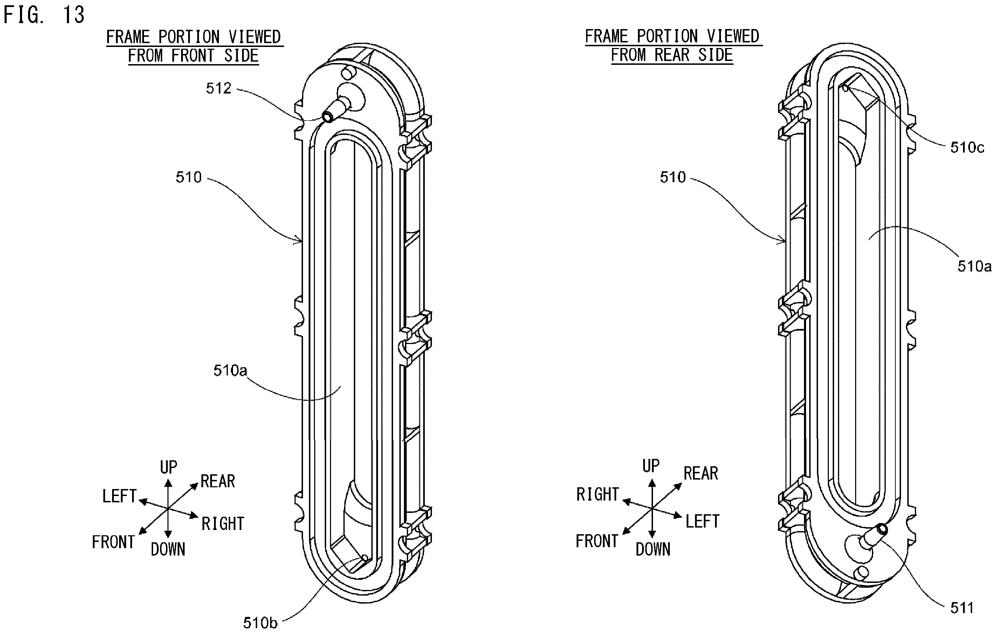

The WDF hemolytic agent to be supplied to the WDF reaction chamber C21, the WDF hemolytic agent to be supplied to the WDF reaction chamber C22, the WNR hemolytic agent to be supplied to the WNR reaction chamber C23, and the RET diluent and the PLT-F diluent to be supplied to the RET/PLT-F reaction chamber C24 are stored in reagent storage portions 501, 502, 503, 504 of a reagent heating unit 500, and are heated. The detailed configuration of the reagent heating unit 500 will be described later with reference to FIGS. 13 to 15.

The outlets 362 of the reaction chambers C21 to C24 are respectively connected to the flow path 651 via valves 611, 621, 631, 641. The syringe pump 652, the valve 653, and the optical measurement part 381 are connected to the flow path 651. A diaphragm pump 655 is connected to the flow path 651 via the valve 653. A valve 654 is connected to the flow path between the valve 653 and the diaphragm pump 655. The disposal ports 363 of the reaction chambers C21 to C24 are respectively connected to a disposal flow path via valves 612, 622, 632, 642.

When preparation of the measurement sample has ended in each reaction chamber shown in FIG. 8, the diaphragm pump 655 draws the prepared measurement sample from the corresponding reaction chamber into the flow path 651. The syringe pump 652 supplies the measurement sample stored in the flow path 651 to the optical measurement part 381. The syringe pump 601 applies a predetermined pressure to the flow path 651 to transfer only a predetermined amount of the measurement sample stored in the flow path 651 to the optical measurement part 381.

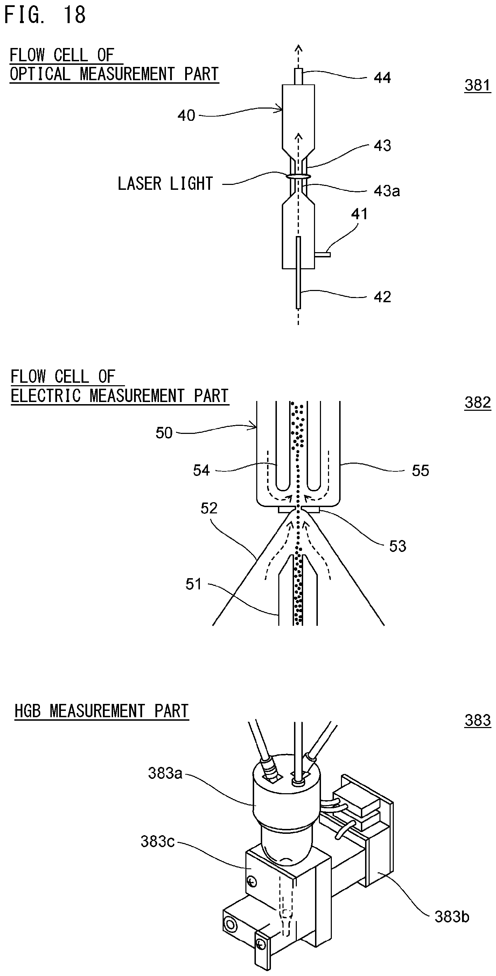

The optical measurement part 381 causes the measurement sample supplied from the flow path 651 and a sheath liquid to flow through a flow cell 40 (see upper part of FIG. 18), and outputs an optical signal corresponding to blood cells in the measurement sample according to a flow cytometry method. The measurement sample having passed through the optical measurement part 381 is discarded. When measurement by the optical measurement part 381 has ended for one measurement sample, a washing liquid is supplied to the reaction chamber in which this measurement sample was prepared. The washing liquid in the reaction chamber is discharged to the flow path 651 through the outlet 362, and is discarded through the disposal port 363. The washing liquid discharged to the flow path 651 is discarded through the valve 654 by the syringe pump 652 and the diaphragm pump 655.

In FIG. 8, the reaction chambers C21 to C24 are connected to the optical measurement part 381 by the common flow path 651. The RET/PLT-F reaction chamber C24 is located at a position closest to the optical measurement part 381 among the plurality of reaction chambers C21 to C24. In other words, the length of the flow path connecting the RET/PLT-F reaction chamber C24 to the optical measurement part 381 is shorter than the lengths of the flow paths connecting the other reaction chambers C21 to C23 to the optical measurement part 381. This design takes into consideration the property of the RET staining liquid used for RET measurement. The RET staining liquid has a property that components tend to remain in the flow path, compared to the other staining liquids. Therefore, in order to minimize influence on the other measurement samples by making the length of the flow path in contact with the measurement sample containing the RET staining liquid as short as possible, the RET/PLT-F reaction chamber C24 is disposed at the position closest to the optical measurement part 381 in the configuration shown in FIG. 8.

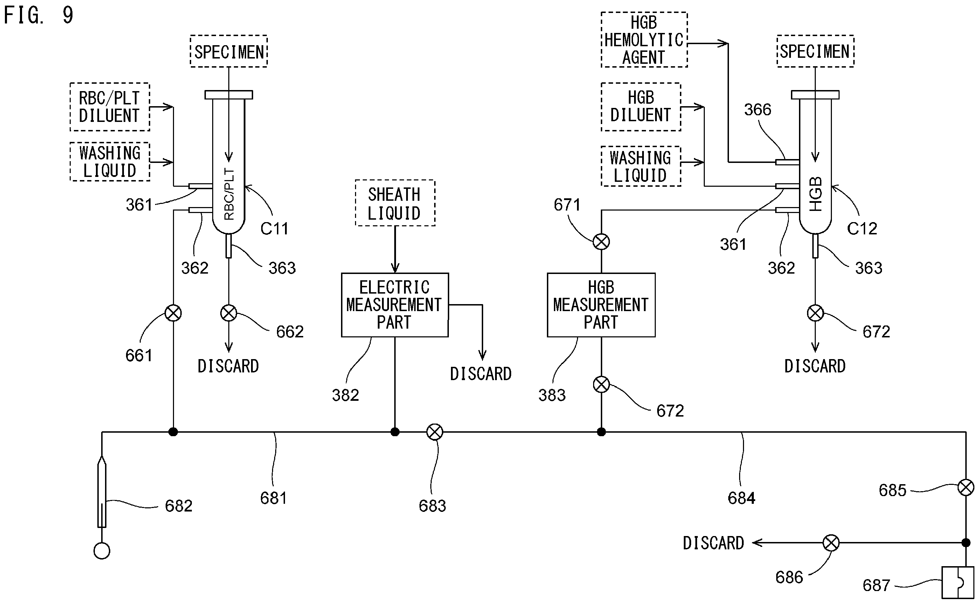

FIG. 9 schematically shows the configuration of a fluid circuit connected to the RBC/PLT reaction chamber C11, the HGB reaction chamber C12, the electric measurement part 382, and the HGB measurement part 383.

The reaction chambers C11, C12 have the same configuration as the above-described reaction chambers C21 to C24. That is, each of the reaction chambers C11, C12 includes an inlet 361 to which a reagent and a washing liquid are supplied, an outlet 362 from which a measurement sample prepared in the reaction chamber is discharged, and a disposal port 363 from which the liquid in the reaction chamber is discharged. However, the HGB reaction chamber C12 further includes an inlet 366 to which a reagent is supplied.

The RBC/PLT diluent and the washing liquid are supplied to the RBC/PLT reaction chamber C11 through the inlet 361. To the HGB reaction chamber C12, the HGB diluent and the washing liquid are supplied through the inlet 361, and the HGB hemolytic agent is supplied through the inlet 366.

The outlet 362 of the RBC/PLT reaction chamber C11 is connected to a flow path 681 through a valve 661. A syringe pump 682, a valve 683, and the electric measurement part 382 are connected to the flow path 681. The outlet 362 of the HGB reaction chamber C12 is connected to the HGB measurement part 383 through a valve 671. The HGB measurement part 383 is connected to a flow path 684 through a valve 672. The valves 683, 685 are connected to the flow path 684. A diaphragm pump 687 is connected to the flow path 684 through the valve 685. A valve 686 is connected to the flow path between the valve 685 and the diaphragm pump 687. The disposal ports 363 of the reaction chambers C11, C12 are respectively connected to the disposal flow path through the valves 662, 672.

When preparation of the RBC/PLT measurement sample has ended in the RBC/PLT reaction chamber C11, the diaphragm pump 687 draws the RBC/PLT measurement sample from the RBC/PLT reaction chamber C11 into the flow path 681. The syringe pump 682 supplies the RBC/PLT measurement sample stored in the flow path 681 to the electric measurement part 382. The syringe pump 682 applies a predetermined pressure to the flow path 681 to transfer only a predetermined amount of the measurement sample stored in the flow path 681, to the electric measurement part 382.

The electric measurement part 382 causes the RBC/PLT measurement sample supplied from the flow path 681 and a sheath liquid to flow through a flow cell 50 (see middle part of FIG. 18), and outputs an electric signal corresponding to blood cells in the RBC/PLT measurement sample according to a sheath flow DC detection method. The RBC/PLT measurement sample having passed through the electric measurement part 382 is discarded. When measurement by the electric measurement part 382 has ended for the RBC/PLT measurement sample, a washing liquid is supplied to the RBC/PLT reaction chamber C11. The washing liquid in the RBC/PLT reaction chamber C11 is discharged to the flow path 681 through the outlet 362, and is discarded through the disposal port 363. The washing liquid discharged to the flow path 681 is discarded through the valve 686 by the syringe pump 682 and the diaphragm pump 687.

When preparation of the HGB measurement sample has ended in the HGB reaction chamber C12, the HGB measurement sample is supplied to the HGB measurement part 383 through the valve 671. The HGB measurement part 383 outputs a signal corresponding to a hemoglobin concentration by an SLS-hemoglobin method, based on the HGB measurement sample. The HGB measurement sample used for the measurement in the HGB measurement part 383 is discarded. When measurement by the HGB measurement part 383 has ended for the HGB measurement sample, a washing liquid is supplied to the HGB reaction chamber C12. The washing liquid in the HGB reaction chamber C12 is discharged to the HGB measurement part 383 through the outlet 362, and is discarded through the disposal port 363. The washing liquid discharged to the HGB measurement part 383 is discharged to the flow path 684 through the inside of the HGB measurement part 383. The washing liquid discharged to the flow path 684 is discarded through the valve 686 by the syringe pump 682 and the diaphragm pump 687.

Next, the configuration of a heating unit 700 will be described with reference to FIGS. 10 to 17.

The heating unit 700 includes the reaction chamber heating unit 400 shown in FIG. 4, and the reagent heating unit 500 shown in FIG. 8. As shown in FIG. 4, the reaction chamber heating unit 400 includes the WDF reaction chambers C21, C22, the WNR reaction chamber C23, and the RET/PLT-F reaction chamber C24.

FIG. 10 is a perspective view showing the configurations of the WDF reaction chambers C21, C22, the WNR reaction chamber C23, and the RET/PLT-F reaction chamber C24.

The reaction chambers C21 to C24 have the same shape, and are formed of the same material. The reaction chambers C21 to C24 are formed of a material having heat resistance, rigidity, and chemical resistance, for example, polyphenylene sulfide (PPS) resin. Each of the reaction chambers C21 to C24 includes an inlet 361, an outlet 362, disposal ports 363, 364, and a trunk portion 365.

The trunk portion 365 is a cylindrical member in which liquid can be stored. The inlet 361, the outlet 362, and the disposal ports 363, 364 are tubular in shape, and are connected to the inside of the trunk portion 365. The disposal port 363 is connected to a lower end of the trunk portion 365 so as to extend downward. The outlet 362 is connected at a position above the connection position of the disposal port 363 so as to extend in the lateral direction with respect to the trunk portion 365. The inlet 361 is connected at a position above the connection position of the outlet 362 so as to extend in the lateral direction with respect to the trunk portion 365. The inlet 361 and the outlet 362 are connected near the lower end of the trunk portion 365.

A discharge outlet 365a opened upward is formed at an upper end of the trunk portion 365. In a plan view, the discharge outlet 365a has a circular shape, and the diameter of the discharge outlet 365a is denoted by R. The discharge outlet 365a is an opening through which, in dispensing a specimen, the lower end portion 351a of the suction tube 351 (see FIG. 6) is moved downward to the inside of the trunk portion 365. The disposal port 364 is connected near the upper end of the trunk portion 365, and prevents the liquid in the trunk portion 365 from flowing over the discharge outlet 365a. The disposal port 364 is connected to the disposal flow path.

The RBC/PLT reaction chamber C11 and the HGB reaction chamber C12 also have the same shape as the reaction chambers C21 to C24, and the HGB reaction chamber C12 further includes an inlet 366 (see FIG. 9). The RBC/PLT reaction chamber C11 and the HGB reaction chamber C12 are formed of, for example, polyvinyl chloride (PVC) resin.

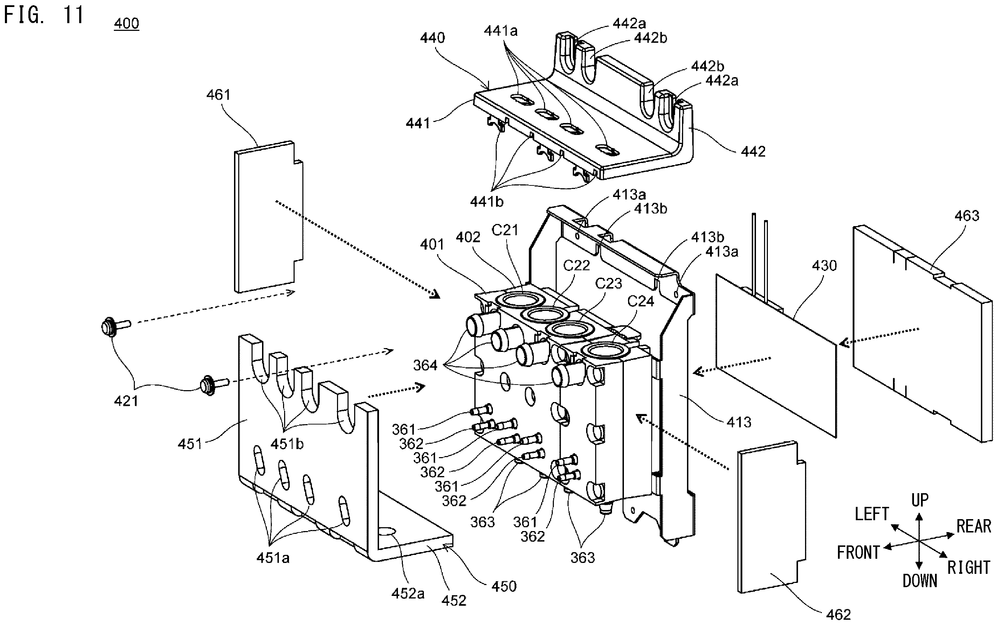

FIG. 11 is an exploded perspective view showing the configuration of the reaction chamber heating unit 400.

The reaction chamber heating unit 400 includes holding blocks 401, 402, a frame member 413, a pair of screws 421, a heater 430, a lid member 440, and heat retaining members 450, 461, 462, 463.

The front-side holding block 401 and the rear-side holding block 402 hold the four reaction chambers C21 to C24 so as to sandwich them from the front and rear sides. The holding blocks 401, 402 are formed of a metal having a high heat conductivity, for example, aluminum. On a rear surface of the holding block 401 and a front surface of the holding block 402, recesses along the outer shapes of the four reaction chambers C21 to C24 are formed. When the holding blocks 401, 402 are joined with the four reaction chambers C21 to C24 being sandwiched therebetween, the four reaction chambers C21 to C24 are housed in the recesses of the holding blocks 401, 402.

The inlets 361, the outlets 362, and the disposal ports 364 of the four reaction chambers C21 to C24 protrude forward from the holding block 401 through holes formed in the holding block 401. The disposal ports 363 of the four reaction chambers C21 to C24 protrude downward from the holding blocks 401, 402 through holes formed in the holding blocks 401, 402.

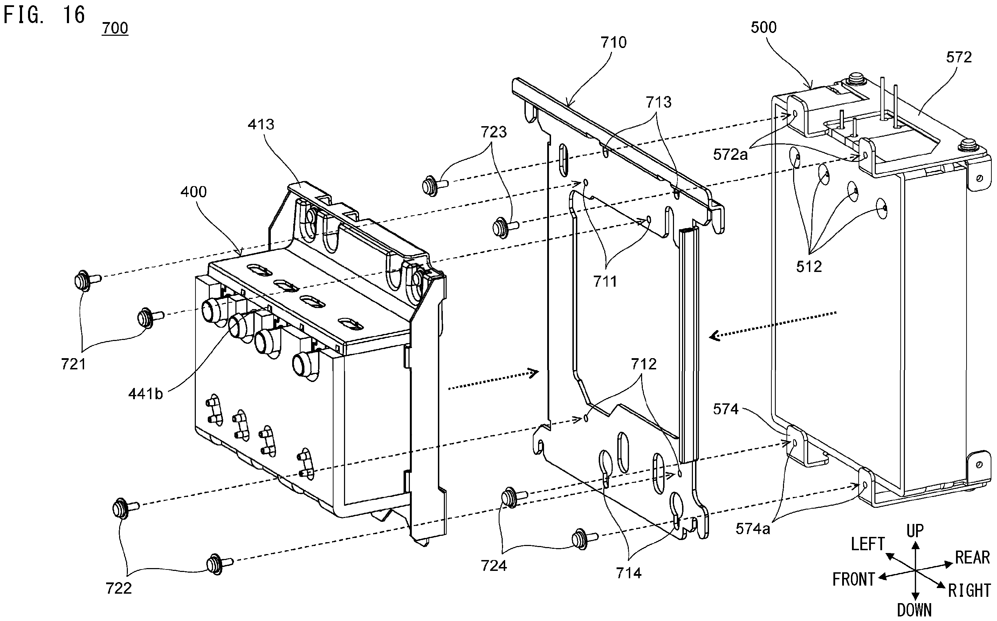

The frame member 413 has a substantially rectangular outer shape, and is disposed on the outer periphery of the rear surface of the holding block 402. A pair of holes 413a for fastening the pair of screws 421 and a pair of holes 413b for fastening a pair of screws 721 (see FIG. 16) described later are formed in the upper side of the frame member 413. In addition, a pair of cutouts for fastening a pair of screws 722 (see FIG. 16) are formed in a lower part of the frame member 413.

The heater 430 is disposed on the rear surface of the holding block 402 via a center opening of the frame member 413 from the rear side of the frame member 413. The heater 430 heats the four reaction chambers C21 to C24. The heater 430 is a sheet heater having a sheet shape. The heater 430 heats the rear surface of the holding block 402 by being electrically driven. The heater 430 is driven to increase the temperature of the liquid in the four reaction chambers C21 to C24 to 35°C to 45°C, more specifically, 41°C. Thus, the measurement samples can be smoothly and appropriately prepared in the four reaction chambers C21 to C24.

The lid member 440 is installed with respect to the upper surfaces of the holding blocks 401, 402. The lid member 440 has an L-shape when viewed from the right, and includes a plate portion 441 parallel to the horizontal surface, and a plate portion 442 perpendicular to the horizontal surface.

The plate portion 441 has four holes 441a penetrating through it in the up-down direction. The four holes 441a are positioned directly above the discharge outlets 365a (see FIG. 10) at the upper ends of the four reaction chambers C21 to C24. Each of the four holes 441a has an elongated-hole shape that is longer in the left-right direction than in the front-rear direction. In dispensing the specimen, the lower end portion 351a of the suction tube 351 (see FIG. 6) is moved downward to the inside of each of the reaction chambers C21 to C24 through the hole 441a.

Four cutouts 441b are formed at the front end of the plate portion 441, and four cutouts corresponding to the four cutouts 441b are also formed at the rear end of the plate portion 441. Tubes extending from the reagent heating unit 500, which are described later, are passed through these cutouts. When the tubes are passed through the cutouts, the tubes pass through the lower surface side of the plate portion 441, which avoids a situation that the tubes are scattered and arranged near the upper part of the plate portion 441 where the lower end portion 351a of the suction tube 351 moves.

A pair of cutouts 442a for fastening a pair of screws 421 and a pair of cutouts 442b for fastening a pair of screws 721 (see FIG. 16) described later, are formed in the plate portion 442. The plate portion 442 is disposed on the upper surfaces of the holding blocks 401, 402, and the pair of screws 421 are fastened in the pair of cutouts 442a of the plate portion 442 and in a pair of holes 413a of the frame member 413. Thus, the lid member 440 is installed with respect to the holding blocks 401, 402 and the frame member 413.

The heat retaining member 450 has an L-shape when viewed from the left, and includes a plate portion 451 perpendicular to the horizontal surface, and a plate portion 452 parallel to the horizontal surface. The heat retaining member 450 is formed of a material having high heat insulation, for example, foamed urethane obtained by adding a foaming agent to polyurethane. The heat retaining member 450 is disposed on the front surface of the holding block 401 and the lower surfaces of the holding blocks 401, 402. In this case, the inlet 361, the outlet 362, and the disposal port 364 provided on the side surfaces of the four reaction chambers C21 to C24 protrude forward from the heat retaining member 450 through holes 451a, 451b formed in the plate portion 451 of the heat retaining member 450. In addition, the disposal ports 363 provided on the lower ends of the four reaction chambers C21 to C24 protrude downward from the heat retaining member 450 through holes 452a formed in the plate portion 452 of the heat retaining member 450.

Each of the heat retaining members 461, 462, 463 has a plate shape perpendicular to the horizontal surface. The heat retaining members 461, 462, 463 are formed of the same material as that of the heat retaining member 450. The heat retaining member 461 is disposed on the left side surfaces of the holding blocks 401, 402, and the heat retaining member 462 is disposed on the right side surfaces of the holding blocks 401, 402. The heat retaining member 463 is disposed on the rear surfaces of the holding block 402 and the heater 430 through the center opening of the frame member 413 from the rear side of the heater 430. Thus, the reaction chamber heating unit 400 is completed.

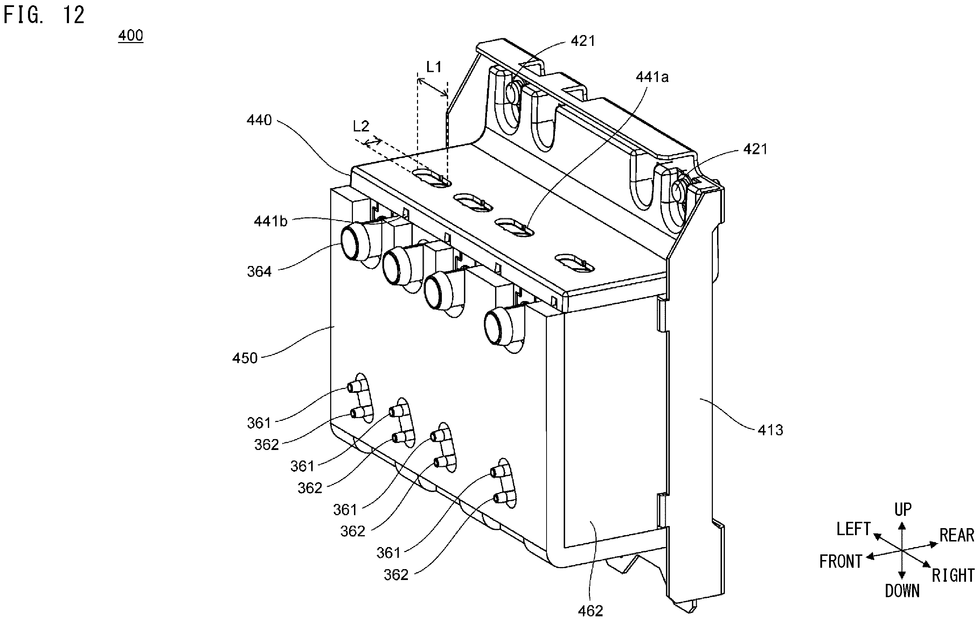

FIG. 12 is a perspective view showing the configuration of the reaction chamber heating unit 400 that has been assembled.

As described above, the lid member 440 has the four holes 441a through which the lower end portion 351a of the suction tube 351 passes. The four holes 441a have the same shape and the same size. A length L1 of each hole 441a in the left-right direction is longer than a length L2 thereof in the front-rear direction. In addition, the length L1 of each hole 441a is equal to a diameter R of the discharge outlet 365a (see FIG. 10) provided at the upper end of each of the reaction chambers C21 to C24. That is, in a plan view, the holes 441a are positioned inside the discharge outlets 365a of the reaction chambers C21 to C24. This prevents dust and the like from entering the reaction chambers C21 to C24 through the holes 441a, while allowing the lower end portion 351a of the suction tube 351 to smoothly pass through the holes 441a.

As shown in FIGS. 11, 12, the front, left, right, and rear surfaces of the reaction chamber heating unit 400 are covered with the heat retaining members 450, 461, 462, 463, respectively, whereby the temperature of the liquid in the four reaction chambers C21 to C24 heated by the heater 430 can be easily kept at 41°C.

FIG. 13 is a perspective view showing the configuration of a frame portion 510 included in the reagent heating unit 500. The frame portion 510 viewed from the front side is shown on the left side in FIG. 13, and the frame portion 510 viewed from the rear side is shown on the right side in FIG. 13.

An opening 510a penetrating the frame portion 510 in the front-rear direction is formed in the center of the frame portion 510. An inlet 511 is disposed on the rear surface near the lower end of the frame portion 510, and an outlet 512 is disposed on the front surface near the upper end of the frame portion 510. The inlet 511 and the outlet 512 are tubular in shape. The inlet 511 communicates with the inside of the opening 510a through a hole 510b formed near the lower end of the opening 510a. The outlet 512 communicates with the inside of the opening 510a through a hole 510c formed near the upper end of the opening 510a.

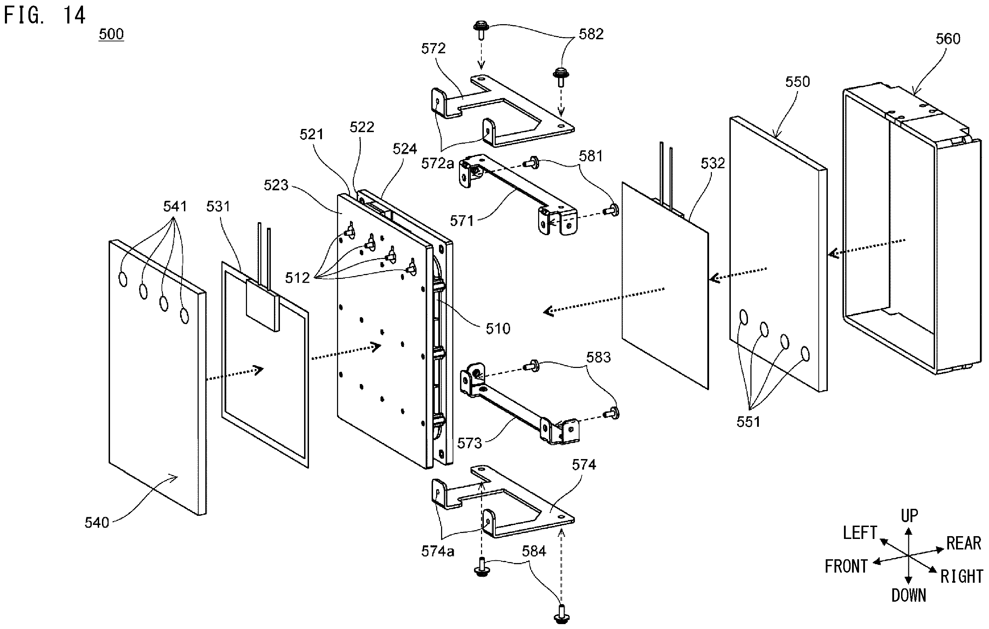

FIG. 14 is an exploded perspective view showing the configuration of the reagent heating unit 500.

The reagent heating unit 500 includes four frame portions 510, sheet members 521, 522, plate members 523, 524, heaters 531, 532, heat retaining members 540, 550, 560, fasteners 571, 572, 573, 574, and screw pairs 581, 582, 583, 584.

The sheet members 521, 522 are formed of a material having heat resistance and chemical resistance, for example, fluororesin. The plate members 523, 524 are formed of a material having heat resistance and rigidity, for example, stainless steel. With the frame portions 510 shown in FIG. 13 being arranged side by side in the left-right direction, the sheet members 521, 522 are disposed so as to sandwich the four frame portions 510 in the front-rear direction. The plate member 523 is disposed on the front surface of the sheet member 521, and the plate member 524 is disposed on the rear surface of the sheet member 522. The plate members 523, 524 are connected to each other at outer peripheral portions thereof. Thus, four sealed spaces are formed by the frame portions 510 and the sheet members 521, 522. The four sealed spaces correspond to the reagent storage portions 501, 502, 503, 504 shown in FIG. 8, respectively.

The heater 531 is disposed on the front surface of the plate member 523, and the heater 532 is disposed on the rear surface of the plate member 524. The heater 531 heats the four reagent storage portions 501 to 504 (see FIG. 8) through the plate member 523 by being electrically driven. The heater 532 heats the four reagent storage portions 501 to 504 through the plate member 524 by being electrically driven. Each of the heaters 531, 532 is a sheet heater having a sheet shape. The heaters 531, 532 are driven so as to heat the liquid in the four reagent storage portions 501 to 504 to 35°C to 45°C, more specifically, 41°C. Thus, the measurement samples can be smoothly and appropriately prepared by using the reagents in the four reaction chambers C21 to C24.

Each of the heat retaining members 540, 550 has a plate shape perpendicular to the horizontal surface. The heat retaining members 540, 550 are formed of a material having high heat insulation, for example, foamed urethane obtained by adding a foaming agent to polyurethane. The heat retaining member 540 is disposed on the front surfaces of the plate member 523 and the heater 531, from the front side of the heater 531. In this case, the outlets 512 of the four frame portions 510 (see left side in FIG. 13) protrude forward from the heat retaining member 540 through holes 541 formed in the heat retaining member 540. The heat retaining member 550 is disposed on the rear surfaces of the plate member 524 and the heater 532, from the rear side of the heater 532. In this case, the inlets 511 of the four frame portions 510 (see right side in FIG. 13) protrude rearward from the heat retaining member 550 through holes 551 formed in the heat retaining member 550.

The heat retaining member 560 is formed of a material having high heat insulation, for example, foamed urethane obtained by adding a foaming agent to polyurethane. The heat retaining member 560 is disposed so as to surround the upper, lower, left, and right sides of the structure obtained by assembling the four frame portions 510, the two sheet members 521, 522, the two plate members 523, 524, the two heaters 531, 532, and the two heat retaining members 540, 550.

The fasteners 571, 572, 573, 574 are members for connecting the reagent heating unit 500 to the reaction chamber heating unit 400. The fastener 571 is disposed near the upper end of the rear surface of the plate member 524 with a pair of screws 581. The fastener 572 is disposed on the upper surface of the fastener 571 with a pair of screws 582. A pair of holes 572a for fastening a pair of screws 723 (see FIG. 16) is formed in the fastener 572. The fastener 573 is disposed near the lower end of the rear surface of the plate member 524 with a pair of screws 583. The fastener 574 is disposed on the lower surface of the fastener 573 with a pair of screws 584. A pair of holes 574a for fastening a pair of screws 724 (see FIG. 16) is formed in the fastener 574. Thus, the reagent heating unit 500 is completed.

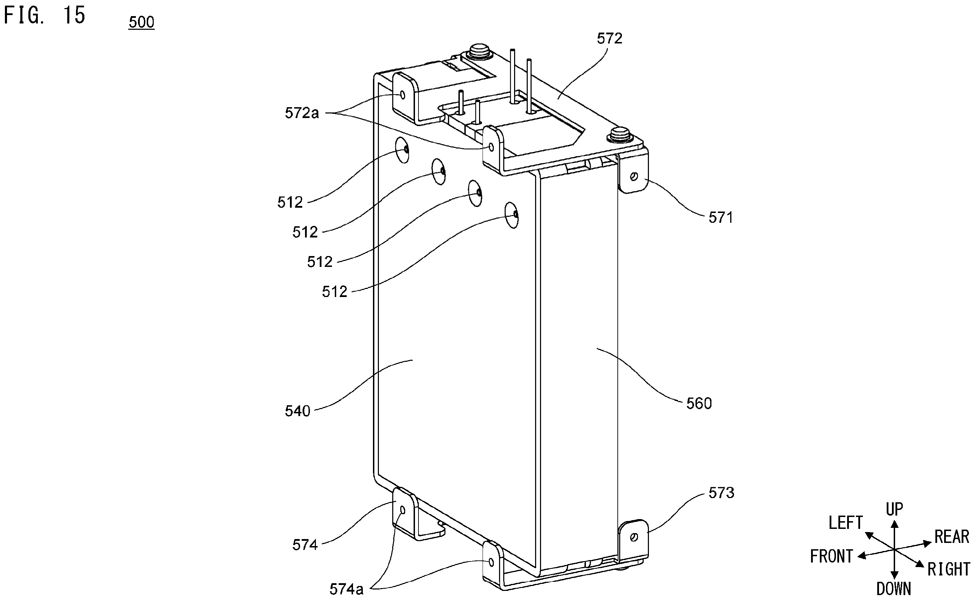

FIG. 15 is a perspective view showing the configuration of the reagent heating unit 500 that has been assembled.

As shown in FIGS. 14, 15, the upper, lower, left, and right surfaces of the reagent heating unit 500 are covered with the heat retaining members 540, 550, 560, whereby the temperature of the reagents in the four reagent storage portions 501 to 504 heated by the heaters 531, 532 can be easily kept at 41°C.

FIG. 16 is an exploded perspective view showing the configuration of the heating unit 700.

The heating unit 700 includes a reaction chamber heating unit 400, a reagent heating unit 500, a fastener 710, and screw pairs 721, 722, 723, 724.

A pair of screws 721 are fastened in a pair of holes 413b of the frame member 413 (see FIG. 11) and a pair of holes 711 of the fastener 710. A pair of screws 722 are fastened in cutouts formed at the lower end of the frame member 413 and holes 712 of the fastener 710. Thus, the reaction chamber heating unit 400 is installed on the fastener 710.

A pair of screws 723 are fastened in a pair of holes 713 of the fastener 710 and a pair of holes 572a of the reagent heating unit 500. A pair of screws 724 are fastened in a pair of holes 714 of the fastener 710 and a pair of holes 574a of the reagent heating unit 500. Thus, the reagent heating unit 500 is installed on the fastener 710.

In assembling the heating unit 700, tubes connected to the four outlets 512 of the reagent heating unit 500 are extended to the front side of the reaction chamber heating unit 400 through the center opening of the fastener 710 and the four cutouts 441b of the reaction chamber heating unit 400. Thus, the heating unit 700 is completed.

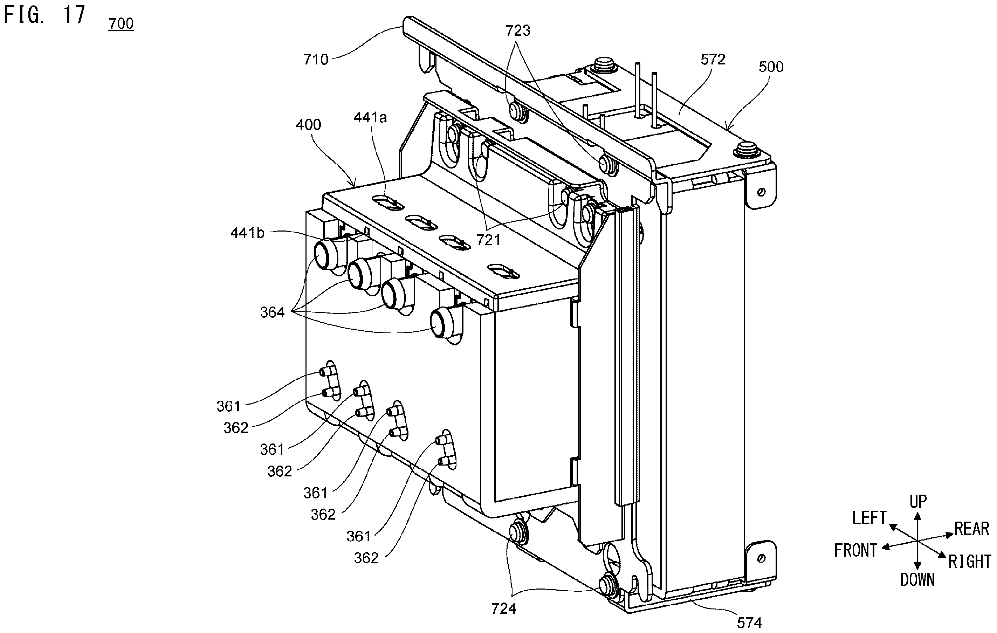

FIG. 17 is a perspective view showing the configuration of the heating unit 700 that has been assembled.

When the heating unit 700 is installed on the measurement unit 10, to the four inlets 511 of the reagent heating unit 500 (see FIG. 13), tubes leading to the reagent containers containing the corresponding reagents are connected. The tubes connected to the four outputs 512 of the reagent heating unit 500 (see left side in FIG. 13) are connected to the inlets 361 of the four reaction chambers C21 to C24 via the four cutouts 441b of the reaction chamber heating unit 400. The outlets 362 of the four reaction chambers C21 to C24 are respectively connected to the flow paths leading to the valves 611, 621, 631, 641 (see FIG. 8). The disposal ports 363 of the four reaction chambers C21 to C24 (see FIG. 10) are respectively connected to the flow paths leading to the valves 612, 622, 632, 642 (see FIG. 8). The disposal ports 364 of the four reaction chambers C21 to C24 are connected to the disposal flow path.

FIG. 18 shows the configuration of the flow cell 40 of the optical measurement part 381, the configuration of the flow cell 50 of the electric measurement part 382, and the configuration of the HGB measurement part 383.

As shown in the upper part of FIG. 18, the flow cell 40 of the optical measurement part 381 includes a sheath liquid supply port 41, a sample nozzle 42, a pore portion 43, and a disposal port 44.

The sheath liquid supply port 41 supplies a sheath liquid into the flow cell 40. The sample nozzle 42 sends out the measurement sample upward in the flow cell 40. The measurement sample advances to the disposal port 44 through a flow path 43a formed in the pore portion 43, while being surrounded by the sheath liquid. The blood cells contained in the measurement sample pass through the flow path 43a in a state of being aligned in one line. The flow path 43a is irradiated with laser light of a predetermined wavelength. When the measurement sample passing through the flow path 43a is irradiated with the laser light, forward scattered light, side scattered light, and fluorescence are generated from the blood cell in the measurement sample. A light receiving portion of the optical measurement part 381 outputs optical signals corresponding to the intensities of the forward scattered light, the side scattered light, and the fluorescence. The intensity of the forward scattered light reflects information regarding the size of the blood cell, the intensity of the side scattered light reflects internal information of the blood cell, and the intensity of the fluorescence reflects the degree of staining of the blood cell. In washing the optical measurement part 381, a washing liquid is supplied to the sheath liquid supply port 41 and the sample nozzle 42.

The optical signal may be a signal that can be obtained as an optical response to irradiation of a blood cell with light. The optical signal is not limited to a signal based on scattered light and a signal based on fluorescence as described above, and may be a signal based on light absorption or a signal based on transmitted light.

As shown in the middle part of FIG. 18, the flow cell 50 of the electric measurement part 382 includes a sample nozzle 51, a chamber 52, an aperture 53, a collection tube 54, and a chamber 55.

The sample nozzle 51 sends out the measurement sample upward. The chamber 52 has a tapered shape that becomes thinner toward the upper side. A sheath liquid is supplied into the chamber 52. The measurement sample advances toward the collection tube 54 through the aperture 53 while being surrounded by the sheath liquid. The blood cells contained in the measurement sample pass the aperture 53 in a state of being aligned in one line. The aperture 53 is provided with electrodes. Direct current is supplied between the electrodes of the aperture 53, and an electric signal according to change in direct current resistance when the measurement sample passes through the aperture 53 is detected. The RBC/PLT diluent used for preparing the RBC/PLT measurement sample contains an electrolyte and therefore has conductivity. As a result, the direct current resistance increases when the blood cell in the RBC/PLT measurement sample passes through the aperture 53, and therefore, the electric signal reflects information on the blood cell passing through the aperture 53.

The sheath liquid is supplied to the chamber 55 so as to flow downward in a region outside the collection tube 54. The sheath liquid flowing outside the collection tube 54 reaches the lower end of the chamber 55 and then flows into the collection tube 54. This prevents the blood cell that has passed through the aperture 53 from returning to the aperture 53, thereby preventing erroneous detection of blood cells. In washing the electric measurement part 382, a washing liquid is supplied to the sample nozzle 51 and the chambers 52, 55.

As shown in the lower part of FIG. 18, the HGB measurement part 383 includes a cell 383a, a light source portion 383b, and a light reception portion 383c.

The cell 383a is formed of a plastic material having high transparency. The light source portion 383b irradiates the cell 383a with light of a wavelength having high absorptivity by SLS-hemoglobin. The light reception portion 383c is disposed opposed to the light source portion 383b with the cell 383a being sandwiched therebetween, and receives transmission light that has been transmitted through the cell 383a.

The HGB measurement sample is stored in the cell 383a. In this state, the light source portion 383b emits light, and the light reception portion 383c receives transmission light. Since the cell 383a is formed of a material having high transparency, the light reception portion 383c receives only transmission light, out of the light emitted from the light source portion 383b, which has not been absorbed by the HGB measurement sample. The light reception portion 383c detects a signal corresponding to the intensity of the transmission light. This signal corresponds to absorbance.

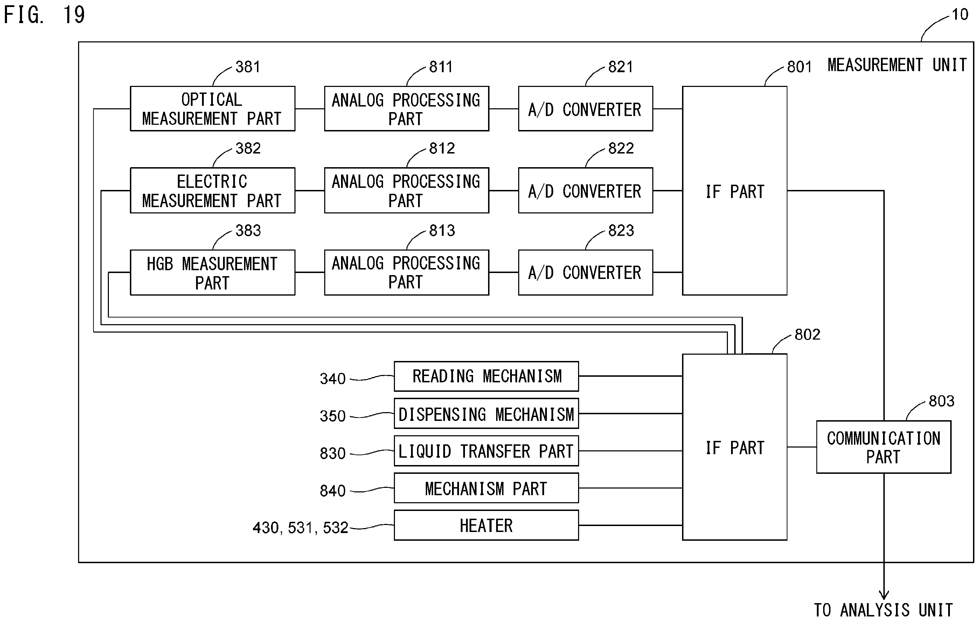

FIG. 19 is a block diagram showing the functional configuration of the measurement unit 10.

The measurement unit 10 includes an optical measurement part 381, an electric measurement part 382, an HGB measurement part 383, analog processing parts 811, 812, 813, A/D converters 821, 822, 823, IF (interface) parts 801, 802, a communication part 803, a reading mechanism 340, a dispensing mechanism 350, a liquid transfer part 830, a mechanism part 840, and heaters 430, 531, 532.

The analog processing parts 811, 812, 813 perform processes such as noise removal and smoothing on analog signals outputted from the optical measurement part 381, the electric measurement part 382, and the HGB measurement part 383, respectively. The A/D converters 821, 822, 823 convert the analog signals processed by the analog processing parts 811, 812, 813, respectively, into digital signals, and transmit the digital signals as measurement results to the analysis unit 30 via the IF part 801 and the communication part 803. The communication part 803 is implemented by a connection terminal based on the USB standard, and performs communication with the analysis unit 30.

The reading mechanism 340 includes a mechanism for driving the rollers 341, 342, and the bar code reader 343. The dispensing mechanism 350 includes the transfer part 353 for transferring the suction tube 351, and the liquid transfer unit 354. The liquid transfer part 830 includes a mechanism for driving the syringe pump, the diaphragm pump, and the valves for transferring the liquid in the flow path shown in FIGS. 8, 9. The mechanism part 840 includes a mechanism for driving the pair of gripping pieces 311 of the gripping mechanism 310, a mechanism for driving the roller 321b of the agitation mechanism 320, a mechanism for rotating the holding part 321 around the rotation shaft 321c, a mechanism for transferring the slide part 322, and a mechanism for transferring the plate member 332 of the container transfer mechanism 330. The respective parts of the measurement unit 10 are controlled by the analysis unit 30 via the IF part 802 and the communication part 803.

FIG. 20 is a block diagram showing the functional configurations of the transport unit 20 and the analysis unit 30.

The transport unit 20 includes the reading mechanism 240, a mechanism unit 251, and the communication part 252.

The reading mechanism 240 includes a mechanism for driving the rollers 241, 242, and a bar code reader 243. The mechanism unit 251 includes a mechanism for driving the pair of members of the transport mechanism 211, a mechanism for driving the conveyor belts 221, 222 of the transport path 220, and a mechanism for driving the member of the transport mechanism 231. The communication part 252 is implemented by a connection terminal based on the USB standard, and performs communication with the analysis unit 30.

The analysis unit 30 includes a controller 850, a memory 861, a display part 31, an input part 32, and communication parts 862, 863.

The controller 850 is implemented by, for example, a CPU. The controller 850 has, as functions, the analysis part 851 for analyzing a specimen and the measurement control part 852 for controlling the measurement unit 10 by performing a computer program stored in the memory 861. The memory 861 is implemented by, for example, an SSD, an HDD, a RAM, or the like. The memory 861 has, stored therein, the measurement result received from the measurement unit 10, a program for controlling the analysis unit 30, and a program for realizing the functions of the analysis part 851 and the measurement control part 852.

The display part 31 is implemented by, for example, a liquid crystal display, an organic EL display, or the like. The input part 32 is implemented by a mouse, a keyboard, and the like. The display part 31 and the input part 32 may be integrally formed as a touch-panel-type display, for example.

The communication part 862 is implemented by a connection terminal based on the USB standard, and performs communication with the communication part 803 of the measurement unit 10 and the communication part 252 of the transport unit 20 via a cable based on the USB standard. The measurement control part 852 controls the respective parts of the measurement unit 10 via the communication part 862, and receives a specimen ID read by the reading mechanism 340 of the measurement unit 10, and the measurement result transmitted from the measurement unit 10. In addition, the measurement control part 852 controls the respective parts of the transport unit 20 via the communication part 862, and receives a specimen ID and a rack ID read by the reading mechanism 240 of the transport unit 20.

The communication part 863 is implemented by a connection terminal based on the Ethernet standard, and performs communication with a host computer outside the analyzer 1 via a cable based on the Ethernet standard. Upon receiving the specimen ID read by the reading mechanism 340 of the measurement unit 10, the measurement control part 852 inquires the host computer of a measurement order via the communication part 863, and receives the measurement order corresponding to the specimen ID from the host computer. The received measurement order for each specimen is stored in the memory 861. In addition, the measurement control part 852 may receive a measurement order from an operator via the input part 32.

The measurement order includes information indicating a predetermined analysis mode such as CBC, DIFF, RET, or PLT-F. For example, the measurement control part 852 controls the respective parts of the measurement unit 10 such that an RBC/PLT measurement sample, an HGB measurement sample, and a WNR measurement sample are prepared when the measurement order is CBC, a WDF measurement sample is prepared in addition to the measurement sample corresponding to CBC when the measurement order is CBC+DIFF, an RET measurement sample is prepared in addition to the measurement samples corresponding to CBC and DIFF when the measurement order is CBC+DIFF+RET, and a PLT-F measurement sample is prepared in addition to the measurement samples corresponding to CBC, DIFF, and RET when the measurement order is CBC+DIFF+RET+PLT-F. Then, the measurement control part 852 causes the optical measurement part 381, the electric measurement part 382, and the HGB measurement part 383 to measure the prepared measurement samples, and obtains the measurement results.

The analysis part 851 analyzes the measurement result of the RBC/PLT measurement sample, and obtains, as analysis results, the number of red blood cells, the number of platelets, a mean corpuscular volume (MCV), etc. The mean corpuscular volume is a measurement value regarding the volume of red blood cells. The analysis part 851 analyzes the measurement result of the HGB measurement sample, and obtains a hemoglobin concentration, etc., as an analysis result. The analysis part 851 classifies the blood cells in the specimen into neutrophils, lymphocytes, monocytes, eosinophils, etc., based on the measurement result of the WDF measurement sample, and classifies the blood cells in the specimen into basophils, etc., based on the measurement result of the WNR measurement sample. Then, the analysis part 851 classifies the blood cells in the specimen into neutrophils, lymphocytes, monocytes, eosinophils, and basophils, based on the classification results of the WDF measurement sample and the WNR measurement sample, and obtains, as analysis results, the numbers of neutrophils, lymphocytes, monocytes, eosinophils, and basophils. The analysis part 851 classifies the blood cells in the specimen into white blood cells, nucleated red blood cells, etc., based on the measurement result of the WNR measurement sample, and obtains, as analysis results, the number of white blood cells, the number of nucleated red blood cells, etc. The analysis part 851 classifies the blood cells in the specimen into reticulocytes, etc., based on the measurement result of the RET measurement sample, and obtains the number of reticulocytes, etc., as an analysis result. The analysis part 851 classifies the blood cells in the specimen into platelets, etc., based on the PLT-F measurement sample, and obtains the number of platelets, etc., as a classification result.

The analysis part 851 may be configured to be able to perform AI analysis according to an artificial intelligence (AI) algorithm, on a measurement result obtained by measuring a specimen. AI analysis allows the analysis part 851 to perform classification of white blood cells (neutrophils, lymphocytes, monocytes, eosinophils, basophils), based on only the measurement result of the WDF measurement sample. In addition, AI analysis allows the analysis part 851 to obtain the number of white blood cells, the number of nucleated red blood cells, etc., based on the measurement result of the WDF measurement sample instead of the measurement result of the WNR measurement sample.

The measurement order may individually include information indicating items of analysis results, in addition to the information indicating the analysis mode as described above. In this case, the measurement control part 852 determines what kind of measurement sample to prepare, based on the items specified in the measurement order. The analysis part 851 analyzes the measurement results obtained from the prepared measurement sample, and obtains the items specified in the measurement order as analysis results.

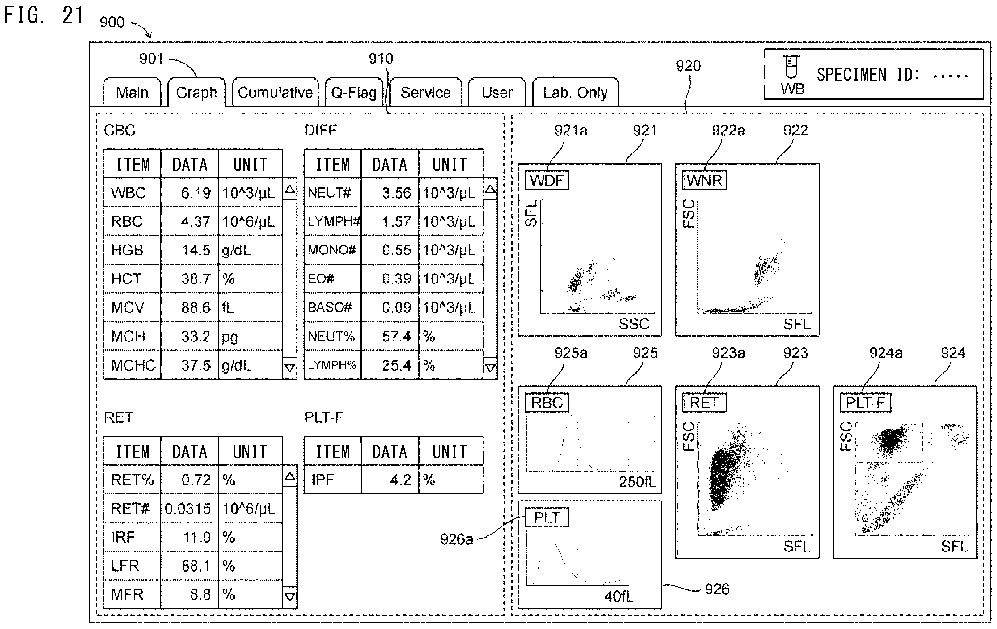

FIG. 21 is a schematic diagram showing the configuration of a screen 900 displayed on the display part 31 of the analysis unit 30.

Upon receiving a screen display instruction from the operator, the controller 850 of the analysis unit 30 displays a screen 900 on the display part 31. The contents displayed on the screen 900 are based on the analysis result generated by the analysis part 851.

The screen 900 includes a plurality of display selection tabs 901 arranged side by side in the horizontal direction, an item display region 910 indicating items of analysis results, and a graph display region 920 indicating graphs of analysis results. The example shown in FIG. 21 indicates a state in which an item display region 910 and a graph display region 920 corresponding to a predetermined specimen ID are displayed because a display selection tab 901 corresponding to "graph" has been operated by the operator.

In the graph display region 920, scattergrams 921, 922, 923, 924 generated based on a WDF measurement sample, a WNR measurement sample, an RET measurement sample, and a PLT-F measurement sample, respectively, are displayed. Also, in the graph display region 920, histograms 925, 926 generated based on an RBC/PLT measurement sample are displayed. In the scattergrams 921 to 924, labels 921a to 926a indicating that the scattergrams are generated based on the WDF measurement sample, the WNR measurement sample, the RET measurement sample, and the PLT-F measurement sample, respectively, are displayed. In the histograms 925, 926, labels 925a, 926a indicating that the histograms are related to red blood cells and platelets generated based on the RBC/PLT measurement sample, are displayed.

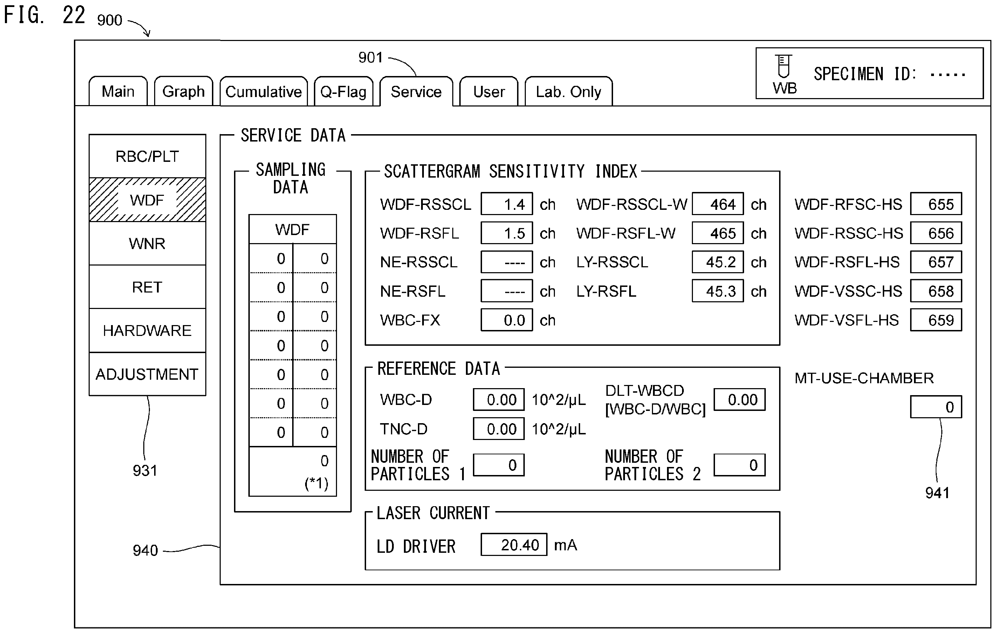

The operator can change the contents displayed on the screen 900 as shown in FIG. 22 by operating the display selection tab 901 corresponding to "service" on the screen 900 shown in FIG. 21.

The screen 900 shown in FIG. 22 includes a plurality of display selection tabs 901 arranged side by side in the horizontal direction, a plurality of display selection buttons 931, and a service data display region 940.

The example shown in FIG. 22 indicates a state in which the display selection button 931 corresponding to "WDF" has been operated by the operator, and various kinds of data regarding the WDF measurement sample are displayed in the service data display region 940. In the service data display region 940 shown in FIG. 22, a display region 941, which numerically indicates which of the WDF reaction chambers C21, C22 has prepared the WDF measurement sample, is displayed. The numerical value in the display region 941 being 0 indicates that the WDF measurement sample has been prepared in the WDF reaction chamber C21, and the numerical value in the display region 941 being 1 indicates that the WDF measurement sample has been prepared in the WDF reaction chamber C22.

The information indicating which of the WDF reaction chambers C21, C22 has prepared the WDF measurement sample may be displayed on the label 921a shown in FIG. 21. For example, when the WDF measurement sample has been prepared in the WDF reaction chamber C21, "WDF-1" may be displayed on the label 921a. When the WDF measurement sample has been prepared in the WDF reaction chamber C22, "WDF-2" may be displayed on the label 921a.

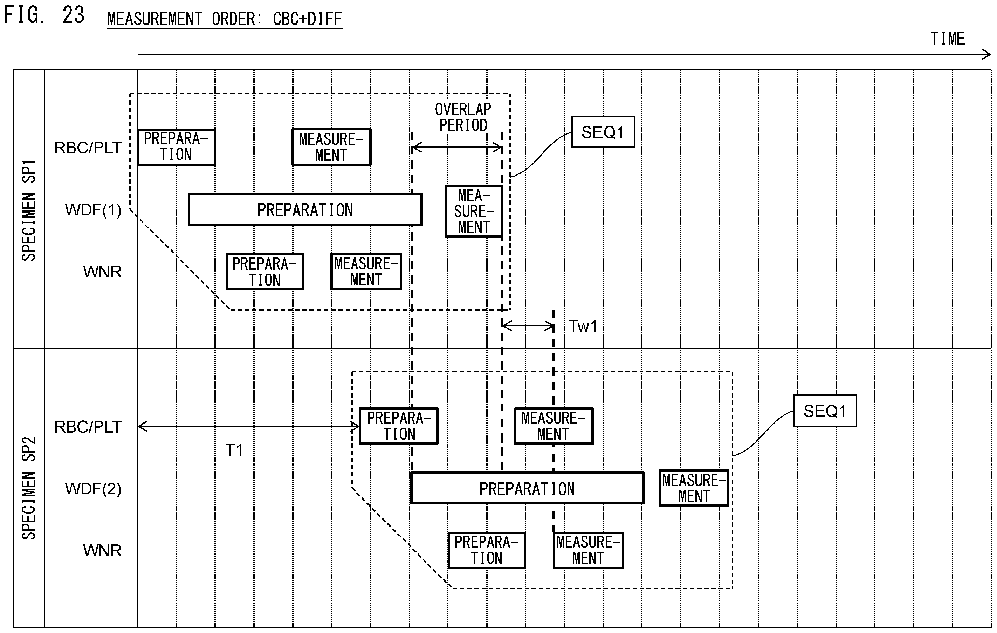

Next, time charts of operations regarding two specimens SP1, SP2 to be consecutively measured will be described with reference to FIGS. 23 to 25.

In the time charts shown in FIGS. 23 to 25, the rightward direction indicates the passage of time, and a time width between two vertical lines adjacent to each other in the left-right direction is 4 seconds. The measurement control part 852 controls preparation of a measurement sample in each reaction chamber and measurement by each measurement part, as shown in the time charts. The measurement control part 852 performs one operation sequence for one specimen. The operation sequence defines the order and timings for operating the components of the measurement unit 10, i.e., the sample preparation parts (i.e., reaction chambers C11, C12, C21 to C24), the measurement parts 381 to 383, and the components such as valves and pumps included in the fluid circuit. When the measurement control part 852 performs the operation sequence, for example, a valve and a pump are controlled such that the valve is opened at a predetermined timing and the pump is driven after a predetermined time. As described later in detail, the measurement control part 852 starts the operation sequence at a predetermined timing to perform, for two consecutive specimens, preparations of WDF measurement samples and measurements by the optical measurement in a partially overlapping manner.

In FIGS. 23 to 25, "RBC/PLT" represents an operation based on an RBC/PLT measurement sample, "WDF(1)" represents an operation based on a measurement sample prepared in the WDF reaction chamber C21, "WDF(2)" represents an operation based on a measurement sample prepared in the WDF reaction chamber C22, "WNR" represents an operation based on a WNR measurement sample, and "RET" represents an operation based on an RET measurement sample. In FIGS. 23 to 25, an operation based on an HGB measurement sample is omitted for convenience.

A rectangular label labeled "preparation" represents a period during which preparation of a measurement sample is performed. Specifically, the left end of the rectangular label of "preparation" indicates a start point of a measurement sample preparation process in the corresponding reaction chamber (timing at which the specimen is discharged into the reaction chamber), and the right end of the rectangular label of "preparation" indicates an end point of the measurement sample preparation process in the corresponding reaction chamber (timing at which transfer of the measurement sample from the reaction chamber is started). A rectangular label labeled "measurement" represents a period during which measurement is performed. Specifically, the left end of the rectangular label of "measurement" indicates a start point of a signal acquisition process in the corresponding measurement unit, and the right end of the rectangular label of "measurement" indicates an end point of the signal acquisition process in the corresponding measurement unit.

The rectangular labels of "preparation" and "measurement" arranged side by side in the horizontal direction indicate a period for preparation and measurement to be performed for one measurement sample, i.e., a processing period corresponding to one measurement sample. A start point of the processing period is the left end of the rectangular label of "preparation", and an end point of the processing period is the right end of the rectangular label of "measurement". Transfer of the measurement sample from the reaction chamber to the measurement unit is performed between the rectangular labels of "preparation" and "measurement".

Specifically, when the measurement sample is transferred to the optical measurement part 381, drawing of the measurement sample by the diaphragm pump 655 and transfer of a predetermined amount of the measurement sample to the optical measurement part 381 by the syringe pump 652 are performed. When the RBC/PLT measurement sample is transferred to the electric measurement part 382, drawing of the RBC/PLT measurement sample by the diaphragm pump 687 and transfer of a predetermined amount of the RBC measurement sample to the electric measurement part 382 by the syringe pump 682 are performed. When the HGB measurement sample is transferred to the HGB measurement part 383, transfer of a predetermined amount of the HGB measurement sample to the HGB measurement part 383 is performed through the valve 671.

When the processing period corresponding to one measurement sample has ended, the corresponding reaction chamber and measurement unit are washed over a predetermined time. When the washing for the predetermined time has ended, the reaction chamber and the measurement unit becomes available.

FIG. 23 is a time chart in a case where a measurement order of CBC+DIFF is set for each of two specimens SP1, SP2 to be consecutively measured.

Upon obtaining a measurement order for the specimen SP1, the measurement control part 852 of the analysis unit 30 specifies an operation sequence based on the obtained measurement order. The memory 861 of the analysis unit 30 has, stored therein, an operation sequence that defines a period of preparation and measurement of a measurement sample for one specimen, according to the type of the measurement order. In the example shown in an upper part of FIG. 23, since the measurement order of the specimen SP1 is CBC+DIFF, the measurement control part 852 drives the measurement unit 10 to read out an operation sequence SEQ1 from the memory 861, and measure the specimen SP1 based on the read operation sequence SEQ1.

When WDF measurement samples of a plurality of specimens are prepared in order, two WDF reaction chambers C21, C22 are alternately used. In the example shown in FIG. 23, the WDF measurement sample of the specimen SP1 is prepared in the WDF reaction chamber C21, and the WDF measurement sample of the specimen SP2 is prepared in the WDF reaction chamber C22.

Once the measurement order is determined, the type of a reaction chamber to be used and the type of a measurement unit to be used are determined. According to the measurement order, the preparation start order of the measurement samples, the preparation time of each measurement sample, the measurement start order of the measurement units, and the measurement time of each measurement unit are determined in advance so as to enable preparation and measurement of the measurement samples. The operation sequence is defined in advance based on the orders, timings, and required times as described above which depend on the measurement order. In addition, the same operation sequence is specified if the measurement order is the same. Thus, variation in measurement result for each measurement is reduced, thereby maintaining the measurement quality.

Since analysis of red blood cells based on the RBC/PLT measurement sample is performed highly frequently, the operation sequence SEQ1 shown in FIG. 23 is defined such that preparation of the RBC/PLT measurement sample is performed before preparation of the WDF measurement sample. In addition, since preparation of the WDF measurement sample requires a longer time than preparation of the WNR measurement sample, the operation sequence SEQ1 shown in FIG. 23 is defined such that preparation of the WDF measurement sample is performed before preparation of the WNR measurement sample.

Subsequently, upon obtaining the measurement order for the specimen SP2, the measurement control part 852 of the analysis unit 30 specifies an operation sequence based on the obtained measurement order. In the example shown in the lower part of FIG. 23 as well, since the measurement order of the specimen SP2 is CBC+DIFF, the measurement control part 852 drives the measurement unit 10 to read out an operation sequence SEQ1 from the memory 861, and measure the specimen SP2 based on the read operation sequence SEQ1.

Here, the optical measurement part 381 is shared by a plurality of sample preparation parts. For example, in the example shown in FIG. 8, the single optical measurement part 381 provided in the measurement unit 10 is shared by a plurality of sample preparation parts (i.e., reaction chambers C21 to C24). Therefore, even if a plurality of measurement samples to be measured by the optical measurement part 381 are prepared in parallel, these measurement samples need to be measured in order by the optical measurement part 381. Therefore, the measurement control part 852 is configured to be able to set a start timing of the operation sequence of the second specimen such that the measurements by the optical measurement part 381 are performed in order even though the periods including measurement sample preparation and measurement of two consecutive specimens (first specimen and second specimen) partially overlap. The start timing is, for example, the earliest timing at which the operation sequence of the second specimen can be started without interference between two consecutive operation sequences. The start timing is set after a predetermined time has elapsed from the start of the operation sequence of the first specimen. The time that defines the start timing is determined depending on the operation sequence of the first specimen.

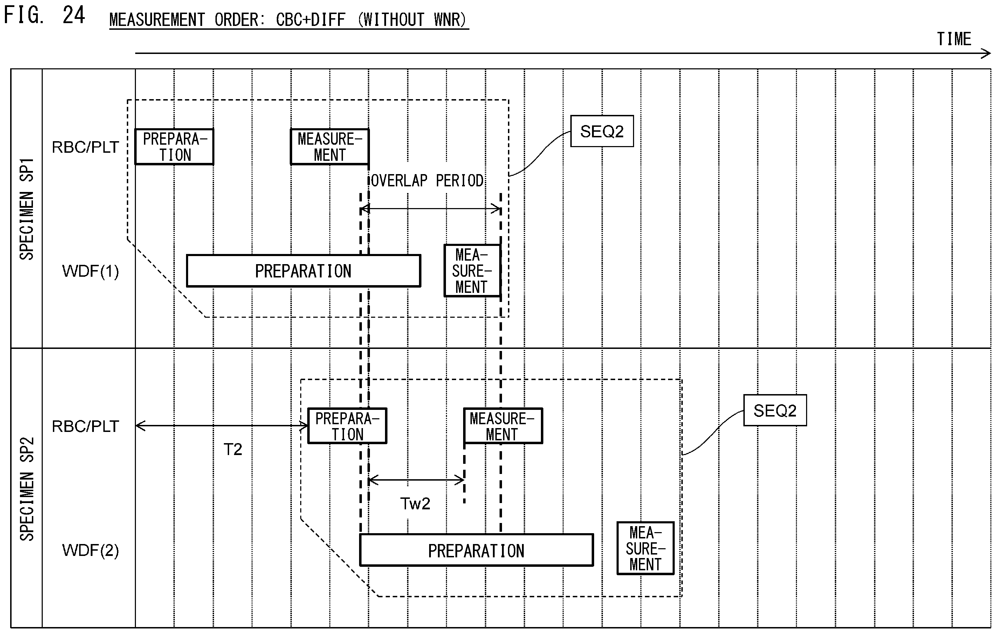

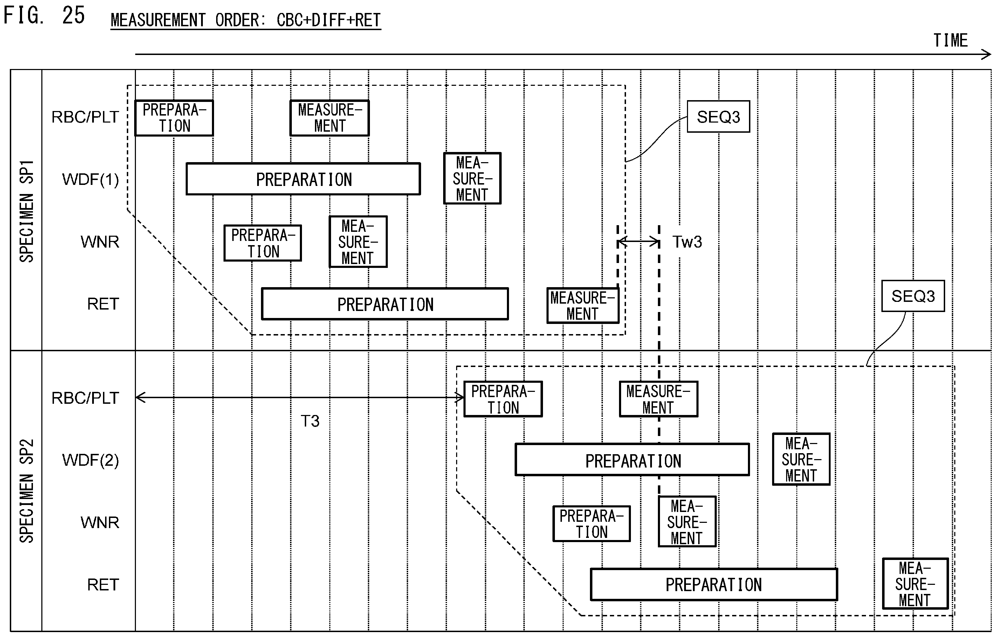

In the example shown in FIG. 23, if the operation sequence of the first specimen SP1 is determined to be SEQ1, the time that defines the start timing of the second specimen SP2 is determined to be T1. Meanwhile, in the examples shown in FIGS. 24, 25 described later, if the operation sequence of the first specimen SP1 is determined to be SEQ2 or SEQ3, the time that defines the start timing of the second specimen SP2 is determined to be T2 or T3, respectively. The time (e.g., any of times T1 to T3) that defines the start timing of the second specimen SP2 is set in advance as a time at which the operation sequence of the second specimen SP2 can be started in parallel with the operation sequence of the first specimen SP1 within a range that does not interfere with the operation sequence of the first specimen SP1.

In the example shown in FIG. 23, time T1 is, for example, a time (1) which is shorter than the time from the start point to the end point of the operation sequence SEQ1 of the first specimen SP1, and (2) in which the measurement processes using the shared optical measurement part 381 in the two operation sequences do not overlap, regardless of the type (e.g., any of SEQ1 to SEQ3) of the operation sequence of the second specimen SP2. In the example shown in FIG. 23, the time T1 is set such that, after the last measurement process for the first specimen SP1 using the optical measurement part 381 (i.e., measurement process for the WDF measurement sample), the first measurement process for the second specimen SP2 using the optical measurement part 381 (i.e., measurement process for the WNR measurement sample) is started with a predetermined waiting time Tw1 in between. The waiting time Tw1 is a time necessary for performing a preparatory operation including, for example, washing of the optical measurement part 381.

As described above, when the measurement order of CBC+DIFF is set for each of the two specimens SP1, SP2 to be consecutively measured, the measurement control part 852 can start the operation sequence of the second specimen SP2 at a start timing when the time T1 has elapsed from the start point of the operation sequence of the first specimen SP1. Thus, a part of the preparation and measurement period regarding the WDF measurement sample of the first specimen SP1 and a part of the preparation and measurement period regarding the WDF measurement sample of the second specimen SP2 can be overlapped with each other in an overlap period shown in FIG. 23. Therefore, preparations and measurements of the two specimens SP1, SP2 can be performed in parallel, thereby improving the throughput of the analyzer 1.

FIG. 24 is a time chart in a case where a measurement order of CBC+DIFF (without WNR) is set for each of the two specimens SP1, SP2 to be consecutively measured. Hereinafter, the differences from the case shown in FIG. 23 will be described.

As described above, when the measurement order is DIFF, classification of white blood cells into five types is usually performed based on the measurement results of the WDF measurement sample and the WNR measurement sample. However, for example, if classification of white blood cells into five types can be performed by AI analysis of the measurement result of the WDF measurement sample, preparation and measurement of the WNR measurement sample need not be performed. FIG. 24 shows an example of an operation sequence in such a case.

Upon obtaining a measurement order for the first specimen SP1, the measurement control part 852 of the analysis unit 30 specifies an operation sequence, based on the obtained measurement order. Since the measurement order of the first specimen SP1 is CBC+DIFF (without WNR) in the example shown in the upper part of FIG. 24, the measurement control part 852 drives the measurement unit 10 to read out the operation sequence SEQ2 from the memory 861, and measure the first specimen SP1 based on the read operation sequence SEQ2.

In response to the operation sequence of the first specimen SP1 being specified to SEQ2, the measurement control part 852 sets a start timing of the second specimen SP2 to a time after time T2 has elapsed from the start point of the operation sequence SEQ2 of the first specimen SP1. The time T2 is, for example, a time (1) which is shorter than the time from the start point to the end point of the operation sequence SEQ2 of the first specimen SP1, and (2) in which the measurement processes using the shared optical measurement part 381 in the two operation sequences do not overlap, regardless of the type (e.g., any of SEQ1 to SEQ3) of the second specimen SP2.

Subsequently, upon obtaining the measurement order for the second specimen SP2, the measurement control part 852 of the analysis unit 30 specifies an operation sequence based on the obtained measurement order. Since the measurement order of the second specimen SP2 is CBC+DIFF (without WNR) also in the example shown in the lower part of FIG. 24, the measurement control part 852 drives the measurement unit 10 to read out the operation sequence SEQ2 from the memory 861, and measure the second specimen SP2 at a start timing after the time T2 has elapsed from the start of the operation sequence of the first specimen SP1, based on the read operation sequence SEQ2.

In FIG. 24 as well, from the viewpoint of the preparatory operation of the optical measurement part 381, when the operation sequences SEQ2 of the two specimens SP1, SP2 are to be performed in parallel, an interval between the measurement end timing of the WDF measurement sample of the first specimen SP1 and the measurement start timing of the WDF measurement sample of the second specimen SP2 can be reduced to the predetermined waiting time Tw1 as in the case shown in FIG. 23. However, in the case shown in FIG. 24, a predetermined waiting time Tw2 longer than the waiting time Tw1 is required between the measurement end timing of the RBC/PLT measurement sample of the first specimen SP1 and the measurement start timing of the RBC/PLT measurement sample of the second specimen SP2. The preparatory operation including, for example, washing of the electric measurement part 382 is performed during such a waiting time Tw2.

As described above, when the measurement order of CBC+DIFF (without WNR) is set for each of the two specimens SP1, SP2 to be consecutively measured, the measurement control part 852 can start the operation sequence of the second specimen SP2 at a start timing when the time T2 has elapsed from the start point of the operation sequence of the first specimen SP1. Thus, a part of the preparation and measurement period regarding the WDF measurement sample of the first specimen SP1 and a part of the preparation and measurement period regarding the WDF measurement sample of the second specimen SP2 can be overlapped with each other in an overlap period shown in FIG. 24. Therefore, preparations and measurements of the two specimens SP1, SP2 can be performed in parallel, thereby improving the throughput of the analyzer 1.

FIG. 25 is a time chart in a case where a measurement order of CBC+DIFF+RET is set for each of the two specimens SP1, SP2 to be consecutively measured. Hereinafter, the differences from the case shown in FIG. 23 will be described.

Upon obtaining the measurement order for the first specimen SP1, the measurement control part 852 of the analysis unit 30 specifies an operation sequence, based on the obtained measurement order. Since the measurement order of the first specimen SP1 is CBC+DIFF+RET in the example shown in the upper part of FIG. 25, the measurement control part 852 drives the measurement unit 10 to read out the operation sequence SEQ3 from the memory 861, and measure the first specimen SP1 based on the read operation sequence SEQ3.

In response to the operation sequence of the first specimen SP1 being specified to the SEQ3, the measurement control part 852 sets a start timing of the second specimen SP2 to a time after time T3 has elapsed from the start point of the operation sequence SEQ3 of the first specimen SP1. The time T3 is, for example, a time (1) which is shorter than the time from the start point to the end point of the operation sequence SEQ3 of the first specimen SP1, and (2) in which the measurement processes using the shared optical measurement part 381 in the two operation sequences do not overlap, regardless of the type (e.g., any of SEQ1 to SEQ3) of the operation sequence of the second specimen SP2.

Subsequently, upon obtaining the measurement order for the second specimen SP2, the measurement control part 852 of the analysis unit 30 specifies an operation sequence based on the obtained measurement order. Since the measurement order of the second specimen SP2 is CBC+DIFF+RET also in the example shown in the lower part of FIG. 25, the measurement control part 852 drives the measurement unit 10 to read out the operation sequence SEQ3 from the memory 861, and measure the second specimen SP2 at a start timing after the time T3 has elapsed from the start of the operation sequence of the first specimen SP1, based on the read operation sequence SEQ3.

In FIG. 25 as well, from the viewpoint of the preparatory operation of the optical measurement part 381, when the operation sequences SEQ3 of the two specimens SP1, SP2 are to be performed in parallel, an interval between the measurement end timing of the RET measurement sample of the first specimen SP1 and the measurement start timing of the WNR measurement sample of the second specimen SP2 can be reduced to a predetermined waiting time Tw3.

As described above, when the measurement order of CBC+DIFF+RET is set for each of the two specimens SP1, SP2 to be consecutively measured, the measurement control part 852 can start the operation sequence of the second specimen SP2 at a start timing when the time T3 has elapsed from the start point of the operation sequence of the first specimen SP1. In this case, although the preparation and measurement periods of the two specimens SP1, SP2 regarding the WDF measurement sample do not overlap each other, preparations and measurements for the two specimens SP1, SP2 can be performed in parallel, thereby improving the throughput of the analyzer 1.

The combination of the operation sequences of the two specimens SP1, SP2 to be consecutively measured is not limited to the combinations shown in FIGS. 23 to 25, and are specified according to the measurement orders of the specimens SP1, SP2. Even if the combination of the operation sequences of the specimens SP1, SP2 is other than those described above, the start timing of the operation sequence of the second specimen SP2 is set according to the type (e.g., any of SEQ1 to SEQ3) of the operation sequence of the first specimen SP1. As described above, the start timing of the second specimen SP2 that depends on the operation sequence of the first specimen SP1 is set to, for example, any of the times T1 to T3, and the times T1 to T3 are times at which the measurement processes using the shared optical measurement part 381 in two operation sequences do not overlap, regardless of the type (e.g., any of SEQ1 to SEQ3) of the operation sequence of the second specimen. Therefore, regardless of the combination of the operation sequences of the specimens SP1, SP2, the measurement samples of the two specimens SP1, SP2 can be measured in this order while sharing the optical measurement part 381.

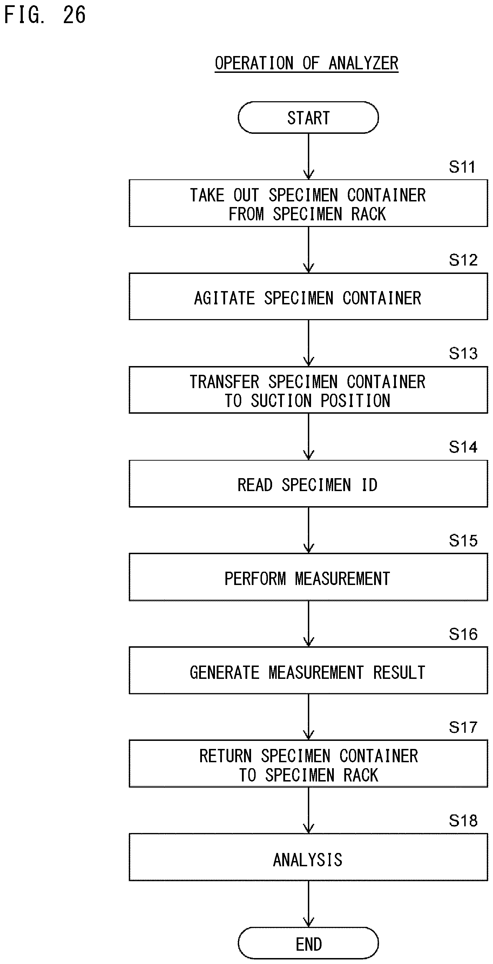

FIG. 26 is a flowchart showing the operation of the analyzer 1 regarding a specimen contained in one specimen container 110.

A process shown in FIG. 26 is repeatedly performed for each specimen. In the following description, one specimen to be subjected to the process shown in FIG. 26 is hereinafter referred to as "current specimen", and one specimen to be subjected to the process shown in FIG. 26 next to the current specimen is hereinafter referred to as "next specimen".

When a specimen container 110 is positioned at a take-out position P1, in step S11, the measurement control part 852 of the analysis unit 30 controls the measurement unit 10 to take out the specimen container 110 at the take-out position P1 from the specimen rack 100. The aforementioned current specimen is contained in the specimen container 110 taken out at step S11. In step S12, the measurement control part 852 controls the measurement unit 10 to agitate the specimen container 110 taken out in step S11. In step S13, the measurement control part 852 controls the measurement unit 10 to transfer the specimen container 110 having been agitated, toward the suction position P4 via the reading position P3.

When the specimen container 110 to be transferred to the suction position P4 is located at the reading position P3, the measurement control part 852 controls the measurement unit 10 to read the bar code from the bar code label 112 of the specimen container 110 at the reading position P3, and obtain the specimen ID of the current specimen in step S14.

In step S15, the measurement control part 852 controls the measurement unit 10 to measure the current specimen inside the specimen container 110 located at the suction position P4. In step S16, the measurement unit 10 generates a measurement result according to the measurement, and transmits the measurement result to the analysis unit 30. The measurement control part 852 stores the received measurement result into the memory 861.

In step S17, the measurement control part 852 controls the measurement unit 10 to return the specimen container 110 to the original hole 101 of the original specimen rack 100. In step S18, the analysis part 851 of the analysis unit 30 performs analysis based on the measurement result obtained in step S16 to obtain an analysis result.

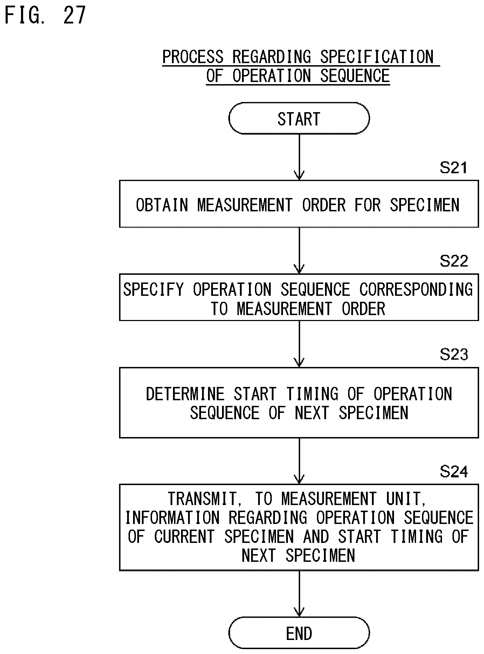

FIG. 27 is a flowchart showing a process of the analysis unit 30 regarding specification of an operation sequence.

The process shown in FIG. 27 is started when the specimen ID of the current specimen has been obtained in step S14 in FIG. 26, and is performed in parallel with the processing shown in FIG. 26. In step S21, the measurement control part 852 of the analysis unit 30 makes an inquiry to the host computer, based on the obtained specimen ID of the current specimen, and obtains a measurement order corresponding to the specimen ID from the host computer.

In step S22, the measurement control part 852 specifies, among a plurality of operation sequences stored in advance in the memory 861, an operation sequence corresponding to the measurement order obtained in step S21, that is, specifies an operation sequence of the current specimen. In step S23, the measurement control part 852 determines a start timing of the operation sequence of the next specimen, based on the operation sequence of the current specimen. Specifically, a time point when a predetermined time (e.g., any of times T1 to T3 shown in FIGS. 23 to 25) corresponding to the operation sequence of the current specimen has elapsed from the start point of the operation sequence of the current specimen is determined as a start timing of the next specimen.

In step S24, the measurement control part 852 transmits, to the measurement unit 10, information regarding the operation sequence of the current specimen specified in step S22 and the start timing of the next specimen determined in step S23. The measurement unit 10 measures the current specimen in steps S 15, S 16 in FIG. 26, based on the received operation sequence of the current specimen.

As described above, in the process of step S23, the start timing of the next specimen is determined based on the operation sequence of the current specimen. Therefore, when the processes shown in FIGS. 26, 27 are performed for the next specimen, the measurement process in step S15 regarding the next specimen (the operation sequence of the next specimen) is started at the start timing determined in step S23 for the current specimen.

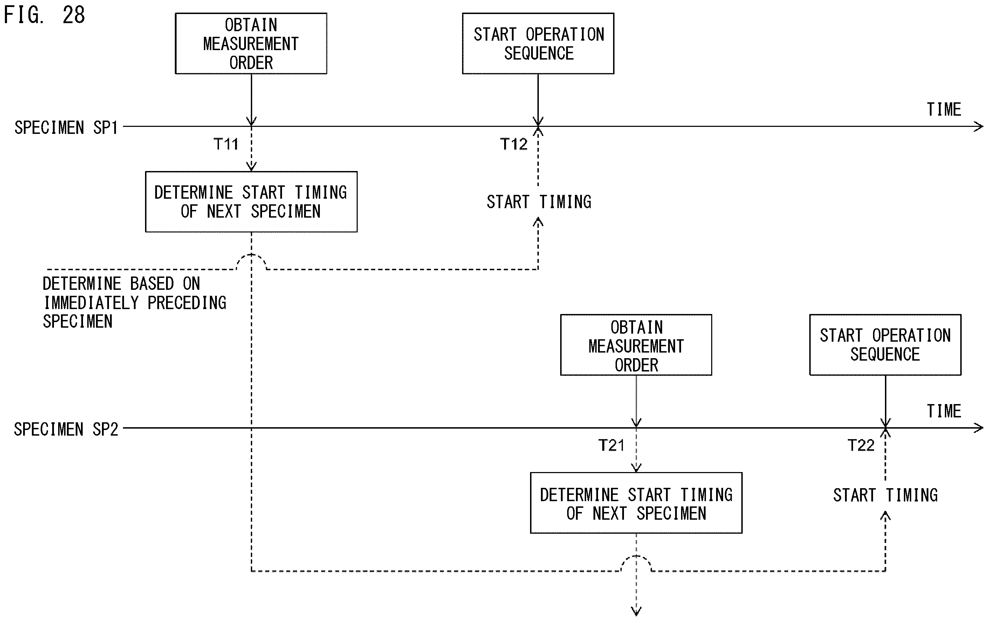

FIG. 28 is a time chart showing an example in which both the operation sequences of the two specimens SP1, SP2 to be consecutively measured are started at start timings.

When the measurement order of the specimen SP1 has been obtained at timing T11, the operation sequence of the specimen SP1 is specified based on the obtained measurement order. Then, the start timing of the operation sequence of the specimen SP2 is determined to be timing T22, based on the operation sequence of the specimen SP1. The start timing of the specimen SP1 is determined based on the specimen immediately preceding the specimen SP1, and is timing T12. When the specimen SP1 is located at the suction position P4 at the start timing T12, the operation sequence of the specimen SP1 is started at the timing T12.

Meanwhile, when the measurement order of the specimen SP2 has been obtained at timing T21, the operation sequence of the specimen SP2 is specified based on the obtained measurement order. Then, the start timing of the operation sequence of the specimen SP2 is determined based on the specified operation sequence of the specimen SP2. The start timing of the specimen SP2 is determined by the specimen SP1, and is the timing T22. When the specimen SP2 is located at the suction position P4 at the start timing T22, the operation sequence of the specimen SP2 is started at the timing T22.

In the example shown in FIG. 28, the measurement order of the specimen SP2 is obtained after the operation sequence of the specimen SP1 has been started. However, the measurement order of the specimen SP2 may be obtained before the start of the operation sequence of the specimen SP1.

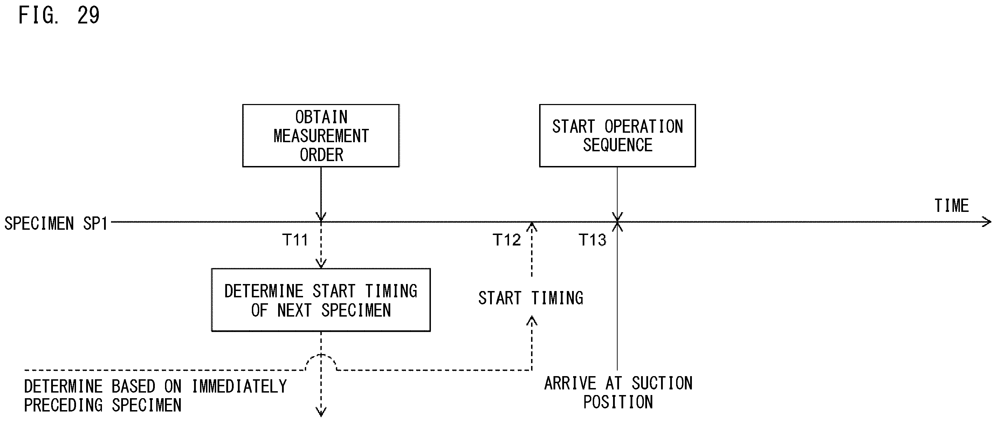

FIG. 29 is a time chart showing an example in which the operation sequence of the specimen SP1 shown in FIG. 28 is not started at the start timing.

In this case as well, the start timing of the specimen SP1 is determined by the specimen immediately preceding the specimen SP1, and is the timing T12. However, depending on, for example, the transfer status of the specimen rack 100 in the transport unit 20, the specimen SP1 may sometimes be located at the suction position P4 at timing T13 after the start timing T12. In this case, the operation sequence of the specimen SP1 is promptly started at the timing T13.

The specimen SP1 may sometimes be located at the suction position P4 before the start timing T12. For example, in the case where the measurement order of the specimen immediately preceding the specimen SP1 is CBC+DIFF+RET+PLT-F and the time required for the operation sequence is long, if the specimen container 110 of the specimen SP1 is taken into the measurement unit 10 subsequently to the specimen container 110 of the immediately preceding specimen, the specimen container 110 of the specimen SP1 waits at the suction position P4 until the start timing T12 arrives. In this case, when the start timing T12 has arrived, the operation sequence of the specimen SP1 is promptly started.

### <Effects of analyzer according to embodiment>

The analyzer 1 includes: the WDF reaction chambers C21, C22 (first and second sample preparation parts) for preparing a WDF measurement sample (first measurement sample) from a specimen by using the WDF hemolytic agent and the WDF staining liquid (first reagent); the RBC/PLT reaction chamber C11 (third sample preparation part) for preparing an RBC/PLT measurement sample (second measurement sample) from the specimen by using the RBC/PLT diluent (second reagent); the optical measurement part 381 that obtains an optical signal corresponding to white blood cells by measuring the WDF measurement sample (first measurement sample); the electric measurement part 382 that obtains an electric signal corresponding to red blood cells by measuring the RBC/PLT measurement sample (second measurement sample); the analysis part 851 that analyzes the specimen, based on the optical signal and the electric signal; and the measurement control part 852 (controller). The measurement control part 852 (controller), according to a plurality of measurement orders, controls preparation of samples in the WDF reaction chambers C21, C22 (first and second sample preparation parts) and measurement of the samples by the optical measurement part 381 such that at least a part of a period including preparation of the WDF measurement sample (first measurement sample) by the WDF reaction chamber C21 (first sample preparation part) and measurement, by the optical measurement part 381, of the WDF measurement sample (first measurement sample) prepared in the WDF reaction chamber C21 (first sample preparation part) overlaps with at least a part of a period including preparation of the WDF measurement sample (first measurement sample) by the WDF reaction chamber C22 (second sample preparation part) and measurement, by the optical measurement part 381, of the WDF measurement sample (first measurement sample) prepared in the WDF reaction chamber C22 (second sample preparation part).

According to this configuration, since the plurality of WDF reaction chambers for preparing WDF measurement samples to be measured by the optical measurement part 381 are provided, at least parts of the periods for sample preparation and measurement can be overlapped with each other. Thus, the throughput of specimen analysis can be improved. In addition, since the electric measurement part is used for RBC measurement, accuracy of RBC measurement can be improved.

As shown in FIGS. 23, 24, the measurement control part 852 (controller) controls preparation of samples in the WDF reaction chambers C21, C22 (first and second sample preparation parts) such that the WDF measurement sample (first measurement sample) is prepared from the specimen SP1 (first specimen) in the WDF reaction chamber C21 (first sample preparation part), and the WDF measurement sample (first measurement sample) is prepared from the specimen SP2 (second specimen) to be measured next to the specimen SP1 (first specimen) in the WDF reaction chamber C22 (second sample preparation part).

According to this configuration, since at least a part of the period including preparation and measurement of the WDF measurement sample based on the specimen SP1 and at least a part of the period including preparation and measurement of the WDF measurement sample based on the specimen SP2 overlap with each other in the overlap period shown in FIGS. 23, 24, analysis of the specimens SP1, SP2 can be quickly advanced.

The measurement control part 852 (controller) controls preparation of samples in the WDF reaction chambers C21, C22 (first and second sample preparation parts) such that, for a plurality of different specimens, preparations of the WDF measurement samples (first measurement samples) in the WDF reaction chambers C21, C22 (first and second sample preparation parts) are alternately performed.

According to this configuration, the WDF measurement samples can be quickly prepared from the plurality of different specimens.

The analyzer further includes a fourth sample preparation part for preparing a third measurement sample from a specimen by using a third reagent. The optical measurement part 381 measures the third measurement sample to further obtain an optical signal corresponding to predetermined blood cells. Here, as a first example, the third reagent is a WNR hemolytic agent and a WNR staining liquid, the third measurement sample is a WNR measurement sample, and the fourth sample preparation part is the WNR reaction chamber C23, and the predetermined blood cells are white blood cells and nucleated red blood cells. As a second example, the third reagent is an RET diluent and an RET staining liquid, the third measurement sample is an RET measurement sample, the fourth sample preparation part is the RET/PLT-F reaction chamber C24, and the predetermined blood cells are reticulocytes. As a third example, the third reagent is a PLT-F diluent and a PLT-F staining liquid, the third measurement sample is a PLT-F measurement sample, the fourth sample preparation part is the RET/PLT-F reaction chamber C24, and the predetermined blood cells are platelets.

According to this configuration, the specimen can be more precisely analyzed based on the optical signal based on the predetermined blood cells.

As shown in FIG. 23, the time for preparing the WDF measurement sample (first measurement sample) is longer than the time for preparing the WNR measurement sample (third measurement sample).

When the time for preparing the WDF measurement sample is longer than the time for preparing the WNR measurement sample, the throughput of the analyzer 1 can be effectively improved by overlapping at least a part of the period based on the WDF reaction chamber C21 with at least a part of the period based on the WDF reaction chamber C22.

The number of WDF reaction chambers (sample preparation parts) for preparing the WDF measurement sample (first measurement sample) is larger than the number of WNR reaction chambers (sample preparation parts) for preparing the WNR measurement sample (third measurement sample).

According to this configuration, the WDF measurement samples corresponding to the respective specimens can be continuously and quickly prepared, compared to preparation of the WNR measurement sample.

As shown in FIG. 23, the measurement control part 852 (controller) controls preparations of samples in either of the WDF reaction chambers C21, C22 (first and second sample preparation parts) and the fourth sample preparation part such that preparation of the WDF measurement sample (first measurement sample) with respect to a specimen is performed before preparation of a third measurement sample with respect to this specimen. Here, as a first example, the third measurement sample is a WNR measurement sample, and the fourth sample preparation part is the WNR reaction chamber C23. As a second example, the third measurement sample is an RET measurement sample, and the fourth sample preparation part is the RET/PLT-F reaction chamber C24. As a third example, the third measurement sample is a PLT-F measurement sample, and the fourth sample preparation part is the RET/PLT-F reaction chamber C24.

According to this configuration, since the WDF measurement sample whose preparation takes time is prepared before the third measurement sample, the processing time for one specimen can be reduced.

The measurement control part 852 (controller) controls preparation of samples in either of the WDF reaction chambers C21, C22 (first and second sample preparation parts) and the fourth sample preparation part such that, for each of a plurality of different specimens, the execution order of preparations of the WDF measurement sample (first measurement sample) and the third measurement sample is the same. Here, as a first example, the third measurement sample is a WNR measurement sample, and the fourth sample preparation part is the WNR reaction chamber C23. As a second example, the third measurement sample is an RET measurement sample, and the fourth sample preparation part is the RET/PLT-F reaction chamber C24. As a third example, the third measurement sample is a PLT-F measurement sample, and the fourth sample preparation part is the RET/PLT-F reaction chamber C24.

According to this configuration, since the execution order of preparations of the WDF measurement sample and the third measurement sample is the same for each specimen, control by the measurement control part 852 can be simplified.

The measurement control part 852 (controller) controls preparations of samples in either of the WDF reaction chambers C21, C22 (first and second sample preparation parts) and the RBC/PLT reaction chamber C11 (third sample preparation part) such that preparation of the RBC/PLT measurement sample (second measurement sample) from a specimen is performed before preparation of the WDF measurement sample (first measurement sample) from this specimen.

In analyzing a specimen by the analyzer 1, analysis of red blood cells based on the RBC/PLT measurement sample is performed highly frequently. According to the above configuration, since preparation of the RBC/PLT measurement sample is performed before preparation of the WDF measurement sample, the sample preparation operation performed on the specimen can be simplified.

The analysis part 851 generates an analysis result including a mean corpuscular volume (MCV, a measurement value regarding the volume of red blood cells), based on an electric signal obtained by the electric measurement part 382.

The accuracy of the analysis result regarding the volume of red blood cells is higher in the case where the analysis result is generated based on the electric signal from the electric measurement part 382 than in the case where the analysis result is generated based on the optical signal from the optical measurement part 381. Therefore, according to the above configuration, a highly accurate analysis result regarding the volume of red blood cells can be obtained.

As shown in the display region 941 in FIG. 22, the analysis part 851 generates the specimen analysis result such that the WDF reaction chamber (sample preparation part) used for preparation of the WDF measurement sample (first measurement sample) is identifiable.

According to this configuration, it is possible to identify which of the WDF measurement samples prepared by the WDF reaction chambers C21 or C22 the generated analysis result is based on. Therefore, for example, when it is determined, based on a measurement result of a quality control substance, that there is a problem in accuracy of the analysis result based on the WDF reaction chamber C21 or C22, it is possible to take a countermeasure such as invalidating the specimen analysis result based on the WDF reaction chamber determined to be problematic.

The measurement control part 852 (controller) controls preparation of samples in the WDF reaction chambers C21, C22 (first and second sample preparation parts) such that the WDF measurement samples (first measurement sample) are prepared under the same preparation conditions.

According to this configuration, since the WDF measurement samples are prepared under the same preparation conditions in the WDF reaction chambers C21, C22, the analysis result is inhibited from varying due to different preparation conditions.

The WDF hemolytic agent and the WDF staining liquid (first reagent) are supplied from a common reagent container to the WDF reaction chambers C21, C22 (first and second sample preparation parts), and the third reagent is supplied from a reagent container, which is different from the reagent containers that contain the WDF hemolytic agent and the WDF staining liquid (first reagent), to the fourth sample preparation part. Here, as a first example, the fourth sample preparation part is the WNR reaction chamber C23, and the third reagent is a WNR hemolytic agent and a WNR staining liquid. As a second example, the fourth sample preparation part is the RET/PLT-F reaction chamber C24, and the third reagent is an RET diluent and an RET staining liquid. As a third example, the fourth sample preparation part is the RET/PLT-F reaction chamber C24, and the third reagent is a PLT-F diluent and a PLT-F staining liquid.

According to this configuration, the WDF measurement samples are prepared in the WDF reaction chambers C21, C22 by using the WDF hemolytic agent stored in the same reagent container and the WDF staining liquid stored in the common reagent container, whereby the preparation conditions for the WDF measurement samples can be shared. This inhibits the analysis result from varying due to different preparation conditions.

The measurement control part 852 (controller) controls the dispensing mechanism 350 (dispenser) such that the same amount of specimen is dispensed to the WDF reaction chambers C21, C22 (first and second sample preparation parts).

According to this configuration, since the same amount of specimen is dispensed to the WDF reaction chambers C21, C22, the preparation conditions for the WDF measurement sample can be shared. This inhibits the analysis result from varying due to different preparation conditions.

The reaction time of the specimen with the WDF hemolytic agent and the WDF staining liquid (first reagent) is common between the WDF reaction chambers C21, C22 (first and second sample preparation parts).

According to this configuration, since the reaction time is common between the WDF reaction chambers C21, C22, the preparation conditions for the WDF measurement sample can be shared. This inhibits the analysis result from varying due to different preparation conditions.

The amount of WDF measurement sample (first measurement sample) to be prepared is common between the WDF reaction chambers C21, C22 (first and second sample preparation parts).

According to this configuration, since the amount of WDF measurement sample to be prepared is common between the WDF reaction chambers C21, C22, the preparation conditions for the WDF measurement sample can be shared. This inhibits the analysis result from varying due to different preparation conditions.

The reaction temperature of the WDF measurement sample (first measurement sample) to be prepared is common between the WDF reaction chambers C21, C22 (first and second sample preparation parts).

According to this configuration, since the reaction temperature of the WDF measurement sample to be prepared is common between the WDF reaction chambers C21, C22, the preparation conditions for the WDF measurement can be shared. This inhibits the analysis result from varying due to different preparation conditions.

The syringe pump 652 (syringe) supplies a predetermined amount of WDF measurement sample (first measurement sample) to the optical measurement part 381. The measurement control part 852 (controller) controls sample preparation and measurement by the optical measurement part 381 in the WDF reaction chambers C21, C22 (first and second sample preparation parts) such that at least a part of a period including preparation of the WDF measurement sample (first measurement sample) by the WDF reaction chamber C21 (first sample preparation part), operation of the syringe pump 652 (syringe), and measurement, by the optical measurement part 381, of the WDF measurement sample (first measurement sample) prepared in the WDF reaction chamber C21 (first sample preparation part), overlaps with at least a part of a period including preparation of the WDF measurement sample (first measurement sample) by the WDF reaction chamber C22 (second sample preparation part), operation of the syringe pump 652 (syringe), and measurement, by the optical measurement part 381, of the WDF measurement sample (first measurement sample) prepared in the WDF reaction chamber C22 (second sample preparation part).

According to this configuration, the amount of the WDF measurement sample supplied from the WDF reaction chamber C21 to the optical measurement part 381 can be made common to the amount of the WDF measurement sample supplied from the WDF reaction chamber C22 to the optical measurement part 381. This inhibits the analysis result from being varied due to different amounts being supplied to the optical measurement part 381.

The syringe pump 652 (syringe) supplies a predetermined amount of WDF measurement sample (first measurement sample) to the optical measurement part 381. The WDF measurement samples (first measurement sample) prepared in the WDF reaction chambers C21, C22 (first and second sample preparation parts) are supplied to the optical measurement part 381 by the common syringe pump 652 (syringe).

According to this configuration, since the WDF measurement samples are supplied from the WDF reaction chambers C21, C22 to the optical measurement part 381 by the common syringe pump 652, the amounts of the WDF measurement samples supplied to the optical measurement part 381 can be made common. This inhibits the analysis result from being varied due to different measurement conditions.

The RBC/PLT reaction chamber C11 (third sample preparation part) is exclusively used for preparation of the RBC/PLT measurement sample (second measurement sample).

According to this configuration, since the RBC/PLT reaction chamber C11 is not used for preparation of other measurement samples, the RBC/PLT measurement sample can be quickly prepared.

The measurement control part 852 (controller) sets the start timing of the second operation sequence corresponding to the second measurement order in which the WDF reaction chamber C22 (second sample preparation part) is used, according to the first operation sequence corresponding to the first measurement order in which the WDF reaction chamber C21 (first sample preparation part) is used.

As shown in FIG. 28, the start timing of the second operation sequence is set based on the first operation sequence. According to this configuration, since the timing to start the second operation sequence is determined when the first operation sequence has been determined, the second operation sequence can be started before the first operation sequence ends. Therefore, at least parts of the periods for preparing and measuring the WDF measurement samples can be overlapped, whereby the effect of improving the throughput of the specimen analysis becomes more remarkable.

The analysis part 851 classifies white blood cells contained in the specimen, based on the optical signal obtained by measuring the WDF measurement sample (first measurement sample).

When classification of white blood cells is performed using the optical signal based on the WDF measurement sample, if the preparation time of the WDF measurement sample is short, the optical signals regarding clusters of the classified white blood cells are close to each other, which makes it difficult to perform appropriate classification. Therefore, it is necessary to ensure a sufficient time for preparing the WDF measurement sample. Even in such a case, the throughput of the specimen analysis can be improved by overlapping at least parts of the periods for sample preparation and measurement as described above with respect to a plurality of specimens.

The optical measurement part 381 is shared by measurement of the measurement sample prepared by the WDF reaction chamber C21 (first sample preparation part) and measurement of the measurement sample prepared by the WDF reaction chamber C22 (second sample preparation part).

According to this configuration, the WDF measurement sample prepared in the WDF reaction chamber C21 and the WDF measurement sample prepared in the WDF reaction chamber C22 cannot be measured in parallel, but the throughput of the specimen analysis can be improved by overlapping the two periods as described above. In addition, since only one optical measurement part 381 is provided, the structure of the analyzer 1 is simplified, and the process for determining whether or not the measurement result based on the optical measurement part 381 is appropriate need not be performed for a plurality of optical measurement parts.

When performing the first operation sequence corresponding to the first measurement order in which the WDF reaction chamber C21 (first sample preparation part) is used and the second operation sequence corresponding to the second measurement order in which the WDF reaction chamber C22 (second sample preparation part) is used, in this order, the measurement control part 852 (controller) sets, as a start timing of the second operation sequence, a start timing which is after the start and before the end of the first operation sequence and which causes measurement of the measurement sample corresponding to the second operation sequence by the optical measurement part 381 to be performed after measurement of the measurement sample corresponding to the first operation sequence by the optical measurement part 381.

Specifically, as shown in FIG. 23, by bringing the measurement end timing of the WDF measurement sample of the specimen SP1 and the measurement start timing of the WNR measurement sample of the specimen SP2 close to the waiting time Tw1, the start timing of the operation sequence of the specimen SP2 is determined to be a timing at which the time T1 has elapsed from the start point of the operation sequence of the specimen SP1. According to this configuration, measurements in the first and second operation sequences can be smoothly performed by using the one optical measurement part 381.

The analyzer 1 is provided with only the WDF reaction chambers C21, C22 (first sample preparation part and the second sample preparation part), as sample preparation parts for preparing the WDF measurement sample (first measurement sample).

According to this configuration, two WDF measurement samples each requiring a long preparation time can be prepared in parallel while inhibiting the structure of the analyzer 1 from being complicated.

The analyzer 1 includes only one RBC/PLT reaction chamber C11 (third sample preparation part), as a sample preparation part for preparing the RBC/PLT measurement sample (second measurement sample).

The time required for preparing the RBC/PLT measurement sample is shorter than the time required for preparing the WDF measurement sample. Therefore, even if only one RBC/PLT reaction chamber C11 is provided, the RBC measurement sample can be smoothly prepared. In addition, providing only one RBC/PLT reaction chamber C11 can inhibit the structure of the analyzer 1 from being complicated.

The analyzer 1 includes: the WDF reaction chambers C21, C22 (first and second sample preparation parts) for preparing the WDF measurement samples (measurement samples) for classifying white blood cells by sharing the WDF hemolytic agent and the WDF staining liquid (white blood cell classification reagents); and the common heater 430 (first heater) for heating the WDF reaction chambers C21, C22 (first and second sample preparation parts).

According to this configuration, the WDF reaction chambers C21, C22 can be heated at substantially equal temperatures by the common heater 430, compared to the case of using separated heaters. Thus, the WDF measurement samples for classifying white blood cells can be prepared under the same temperature condition in the WDF reaction chambers C21, C22. Therefore, the analysis result is inhibited from being varied due to different temperature conditions. In addition, the common heater 430 being arranged simplifies the structure of the analyzer 1.

The WDF reaction chambers C21, C22 (first and second sample preparation parts) are arranged adjacent to each other.

According to this configuration, in dispensing the specimen to each of the WDF reaction chambers C21, C22, the transfer paths of the suction tube 351 that dispenses the specimen can be brought close to each other, thereby simplifying the transfer control for the suction tube 351.

The analyzer 1 further includes the WNR reaction chamber C23 (third sample preparation part) for preparing the WNR measurement sample (measurement sample) to be used for obtaining the number of white blood cells. The heater 430 (first heater) is provided to be shared by the WDF reaction chambers C21, C22 and the WNR reaction chamber C23 (first, second, and third sample preparation parts).

If the temperature condition for the WNR measurement sample to be used for obtaining the number of white blood cells is the same as the temperature condition for the WDF measurement sample to be used for classifying white blood cells, the above configuration allows the WDF reaction chambers C21, C22 and the WNR reaction chamber C23 to be heated by the common heater 430 at substantially equal temperatures. Thus, in the respective chambers, the measurement samples can be prepared under uniform temperature conditions. In addition, since the common heater 430 is provided, the structure of the analyzer 1 can be simplified.

The analyzer 1 further includes the RET/PLT-F reaction chamber C24 (fourth sample preparation part) for preparing the RET measurement sample (measurement sample) used for classifying reticulocytes. The heater 430 (first heater) is provided to be shared by the WDF reaction chambers C21, C22 and the RET/PLT-F reaction chamber C24 (first, second, and fourth sample preparation parts).

If the temperature condition for the RET measurement sample to be used for classifying the reticulocytes is the same as the temperature condition for the WDF measurement sample to be used for classifying white blood cells, the above configuration allows the WDF reaction chambers C21, C22 and the RET/PLT-F reaction chamber C24 to be heated by the common heater 430 at substantially equal temperatures. Thus, in the respective chambers, the measurement samples can be prepared under uniform temperature conditions. In addition, since the common heater 430 is provided, the structure of the analyzer 1 can be simplified.

The analyzer 1 further includes the suction tube 351 for suctioning the specimen from the specimen container 110 at the suction position P4, and dispensing the suctioned specimen. The WDF reaction chambers C21, C22 and the RET/PLT-F reaction chamber C24 (first, second, and fourth sample preparation parts) are arranged closer to the suction position P4 in this order.

Classification of reticulocytes is less frequent than classification of white blood cells. Therefore, the above configuration in which the WDF reaction chambers C21, C22 are arranged closer to the suction position P4 than the RET/PLT-F reaction chamber C24 (fourth sample preparation part) allows specimen dispensing to be smoothly performed.

The heater 430 (first heater) is a sheet heater.

According to this configuration, the heater 430 can be compactly disposed in the analyzer 1.

The heater 430 (first heater) heats the liquid in the WDF reaction chambers C21, C22 (first and second sample preparation parts) to 35°C to 45°C.

According to this configuration, in the WDF reaction chambers C21, C22, the WDF measurement sample can be smoothly prepared.

The WDF reaction chambers C21, C22 (first and second sample preparation parts) are connected to the same reagent container.

According to this configuration, the measurement sample preparation conditions can be made more uniform in the WDF reaction chambers C21, C22.

The analyzer 1 further includes the common heaters 531, 532 (second heater) for heating the WDF hemolytic agent (reagent) supplied to the WDF reaction chambers C21, C22 (first and second sample preparation parts).

According to this configuration, the common heaters 531, 532 can heat the WDF hemolytic agent to be supplied to the WDF reaction chambers C21, C22 at substantially equal temperatures, compared to the case where the WDF reaction chambers C21, C22 are provided with individual heaters. Thus, the WDF hemolytic agent of a uniform temperature can be supplied to each of the WDF reaction chambers C21, C22. Therefore, the analysis result is inhibited from being varied due to different temperature conditions for the WDF hemolytic agent. In addition, since the common heaters 531, 532 are provided, the structure of the analyzer 1 can be simplified.

The analyzer 1 further includes the dispensing mechanism 350 that suctions the specimen from the specimen container 110 through the suction tube 351, and dispenses the suctioned specimen to the WDF reaction chambers C21, C22 (first and second sample preparation parts). The suction position P4 at which the specimen is suctioned from the specimen container 110 and the discharge outlet 365a of the WDF reaction chambers C21, C22 (first and second sample preparation parts) are arranged side by side in a straight line.

According to this configuration, it is possible to simplify the structure and control of the dispensing mechanism 350 that transfers the suction tube 351 to the suction position P4 and the discharge outlets 365a of the WDF reaction chambers C21, C22.

The analyzer 1 further includes the RBC/PLT reaction chamber C11 (fifth sample preparation part) for preparing the RBC/PLT measurement sample (measurement sample) used for obtaining the number of red blood cells and the number of platelets. The dispensing mechanism 350 further dispenses, into the RBC/PLT reaction chamber C11 (fifth sample preparation part), the specimen suctioned from the specimen container 110 through the suction tube 351. The discharge outlets 365a of the WDF reaction chambers C21, C22 and the RBC/PLT reaction chamber C11 (first, second, and fifth sample preparation parts) are arranged side by side in a straight line.

According to this configuration, it is possible to further simplify the structure and control of the dispensing mechanism 350 that transfers the suction tube 351 to the discharge outlets 365a of the WDF reaction chambers C21, C22 and the RBC/PLT reaction chamber C11.

The analyzer 1 further includes the HGB reaction chamber C12 (sixth sample preparation part) for preparing the HGB measurement sample (measurement sample) to be used for obtaining hemoglobin concentration. The dispensing mechanism 350 further dispenses, into the HGB reaction chamber C12 (sixth sample preparation part), the specimen suctioned from the specimen container 110 through the suction tube 351. The discharge outlets 365a of the WDF reaction chambers C21, C22 and the HGB reaction chamber C12 (first, second, and sixth sample preparation parts) are arranged side by side in a straight line.

According to this configuration, it is possible to further simplify the structure and control of the dispensing mechanism 350 that transfers the suction tube 351 to the discharge outlets 365a of the WDF reaction chambers C21, C22 and the HGB reaction chamber C12.

### <Modifications>

In the above embodiment, an operation sequence of a specimen is determined based on a measurement order set for the specimen. However, if the measurement order to be set for the specimen has been determined in advance, the operation sequence of the specimen may be determined to be a predetermined operation sequence. For example, if the analyzer 1 performs measurement and analysis of the specimen with the measurement order being always CBC+DIFF, the operation sequence of the specimen is always determined to be the operation sequence SEQ1. In this case, as shown in FIG. 23, the operation sequences SEQ1 of the two consecutive specimens SP1, SP2 are performed so as to overlap with each other.

In the above embodiment, the two consecutive specimens SP1, SP2 are not limited to specimens collected from different subjects, but may also be specimens collected from the same subject at different timings, or specimens into which a specimen collected from the same subject at a predetermined timing is divided.

In the above embodiment, two WDF reaction chambers are provided, but three or more WDF reaction chambers may be provided. The RET/PLT-F reaction chamber C24 may be configured to be divided into an RET reaction chamber for preparing an RET measurement sample, and a PLT-F reaction chamber for preparing a PLT-F measurement sample.

In the above embodiment, the diaphragm pump 655 is used for drawing liquid from the reaction chambers C21 to C24 into the flow path 651, but another mechanism may be used. The diaphragm pump 687 is used for drawing liquid from the RBC/PLT reaction chamber C11 and the HGB measurement part 383 into the flow paths 681, 684, but another mechanism may be used. The syringe pump 652 may have a function of drawing liquid from the reaction chambers C21 to C24 into the flow path 651. The syringe pump 682 may have a function of drawing liquid from the RBC/PLT reaction chamber C11 and the HGB measurement part 383 into the flow paths 681, 684.

In the above embodiment, as shown in FIG. 4, the suction position P4 and the discharge outlets of the reaction chambers C11, C12, C21 to C24 are arranged side by side in a straight line, but may be arranged in a polygonal line or a curved line. However, from the viewpoint of simplifying the structure and control of the dispensing mechanism 350 for transferring the suction tube 351, the suction position P4 and the discharge outlets of the reaction chambers C11, C12, C21 to C24 are preferably arranged side by side in a straight line.

In the above embodiment, as shown in FIG. 11, the intervals between the reaction chambers C21 to C24 are not constant, and the interval between the reaction chambers C23, C24 is slightly larger than the other intervals. However, the reaction chambers C21 to C24 may be arranged at constant intervals. Thus, control of the dispensing mechanism 350 that transfers the suction tube 351 can be simplified.

In addition to the above, various modifications of the embodiment of the present invention may be made as appropriate without departing from the scope of the technical idea defined by the claims.

### [Remarks]

The present disclosure includes the following items 1-27.

Item 1: An analyzer configured to analyze a specimen, comprising:
first and second sample preparation parts configured to prepare a first measurement sample from the specimen by using a first reagent;
a third sample preparation part configured to prepare a second measurement sample from the specimen by using a second reagent;
an optical measurement part configured to measure the first measurement sample to obtain an optical signal corresponding to white blood cells;
an electric measurement part configured to measure the second measurement sample to obtain an electric signal corresponding to red blood cells; and
a controller programmed to analyze the specimen, based on the optical signal and the electric signal, wherein
the controller is further programmed to
control, according to a plurality of measurement orders, preparation in the first and second sample preparation parts and measurement by the optical measurement part such that at least a part of a first period overlaps with at least a part of a second period,
the first period including a preparation of the first measurement sample by the first sample preparation part and a measurement thereof by the optical measurement part, and
the second period including a preparation of the first measurement sample by the second sample preparation part and a measurement thereof by the optical measurement part.

Item 2: The analyzer of item 1, wherein
the controller is further programmed to control sample preparation in the first and second sample preparation parts such that
the first measurement sample is prepared from the first specimen in the first sample preparation part, and
the first measurement sample is prepared from a second specimen to be measured in the second sample preparation part next to the first specimen.

Item 3: The analyzer of item 1, wherein
the controller is further programmed to control sample preparation in the first and second sample preparation parts such that, for a plurality of different specimens, preparations of first measurement samples in the first and second sample preparation parts are alternately performed.

Item 4: The analyzer of item 1, further comprising a fourth sample preparation part configured to prepare a third measurement sample from the specimen by using a third reagent, wherein
the optical measurement part measures the third measurement sample to further obtain an optical signal corresponding to predetermined blood cells.

Item 5: The analyzer of item 4, wherein
a time for preparing the first measurement sample is longer than a time for preparing the third measurement sample.

Item 6: The analyzer of item 5, wherein
the number of sample preparation parts for preparing the first measurement sample is larger than the number of sample preparation parts for preparing the third measurement sample.

Item 7: The analyzer of item 4, wherein
the controller is further programmed to control sample preparation in either of the first and second sample preparation parts and the fourth sample preparation part such that preparation of the first measurement sample with respect to the specimen is performed before preparation of the third measurement sample with respect to the specimen.

Item 8: The analyzer of item 4, wherein
the controller is further programmed to control sample preparation in either of the first and second sample preparation parts and the fourth sample preparation part such that, for each of a plurality of different specimens, the order of preparations of the first and third measurement samples is the same.

Item 9: The analyzer of item 1, wherein
the controller is further programmed to control sample preparation in either of the first and second sample preparation parts and the third sample preparation part such that preparation of the second measurement sample with respect to the specimen is performed before preparation of the first measurement sample with respect to the specimen.

Item 10: The analyzer of item 1, wherein
the analysis part generates an analysis result including a measured value regarding a volume of red blood cells, based on the electric signal.

Item 11: The analyzer of item 1, wherein
the analysis part generates an analysis result of the specimen such that a sample preparation part used for preparing the first measurement sample becomes identifiable.

Item 12: The analyzer of item 1, wherein
the controller is further programmed to control sample preparation in the first and second sample preparation parts such that the first measurement samples are prepared with common preparation conditions.

Item 13: The analyzer of item 1, wherein
the first reagent is supplied to the first and second sample preparation parts from a common reagent container, and
the third reagent is supplied to the fourth sample preparation part from a reagent container different from the reagent container that contains the first reagent.

Item 14: The analyzer of item 1, further comprising a dispenser configured to dispense the specimen, wherein
the controller is further programmed to control the dispenser such that the same amount of specimen is dispensed to the first and second sample preparation parts.

Item 15: The analyzer of item 1, wherein
a reaction time of the specimen with the first reagent is common between the first and second sample preparation parts.

Item 16: The analyzer of item 1, wherein
the amount of the first measurement sample to be prepared is common between the first and second sample preparation parts.

Item 17: The analyzer of item 1, wherein
a reaction temperature of the first measurement sample to be prepared is common between the first and second sample preparation parts.

Item 18: The analyzer of item 1, further comprising a syringe configured to supply a predetermined amount of the first measurement sample to the optical measurement part, wherein
the controller is further programmed to control sample preparation in the first and second sample preparation parts and sample measurement by the optical measurement part such that
at least a part of a period including preparation of the first measurement sample in the first sample preparation part, operation of the syringe, and measurement, by the optical measurement part, of the first measurement sample prepared in the first sample preparation part overlaps with at least a part of a period including preparation of the first measurement sample in the second sample preparation part, operation of the syringe, and measurement, by the optical measurement part, of the first measurement sample prepared in the second sample preparation part.

Item 19: The analyzer of item 1, further comprising a syringe configured to supply a predetermined amount of the first measurement sample to the optical measurement part, wherein
the first measurement samples prepared in the first and second sample preparation parts are supplied to the optical measurement part through the syringe that is shared.

Item 20: The analyzer of item 1, wherein
the third sample preparation part is exclusively used for preparation of the second measurement sample.

Item 21: The analyzer of item 1, wherein
the controller sets a start timing of a second operation sequence corresponding to a second measurement order in which the second sample preparation part is used, according to a first operation sequence corresponding to a first measurement order in which the first sample preparation part is used.

Item 22: The analyzer of item 1, wherein
the analysis part classifies white blood cells contained in the specimen, based on the optical signal obtained by measurement of the first measurement sample.

Item 23: The analyzer of item 1, wherein
the optical measurement part is shared by measurement of a measurement sample prepared by the first sample preparation part, and measurement of a measurement sample prepared by the second sample preparation part.

Item 24: The analyzer of item 23, wherein
when performing a first operation sequence corresponding to a first measurement order in which the first sample preparation part is used and a second operation sequence corresponding to a second measurement order in which the second sample preparation part is used, in this order, the controller sets, as a start timing of the second operation sequence, a start timing which is after start and before end of the first operation sequence and which causes measurement of the measurement sample corresponding to the second operation sequence by the optical measurement part to be performed after measurement of the measurement sample corresponding to the first operation sequence by the optical measurement part.

Item 25: The analyzer of item 1, wherein
only the first sample preparation part and the second sample preparation part are provided as sample preparation parts for preparing the first measurement sample.

Item 26: The analyzer of item 25, wherein
only a single third sample preparation part is provided as a sample preparation part for preparing the second measurement sample.

Item 27: The analyzer of item 1, further comprising a common heater configured to heat the first sample preparation part and the second sample preparation part.

### DESCRIPTION OF THE REFERENCE CHARACTERS

1 analyzer
350 dispensing mechanism (dispenser)
381 optical measurement part
382 electric measurement part
430 heater
652 syringe pump (syringe)
851 analysis part
852 measurement control part (controller)
C11 RBC/PLT reaction chamber (third sample preparation part)
C21 WDF reaction chamber (first sample preparation part)
C22 WDF reaction chamber (second sample preparation part)
C23 WNR reaction chamber (fourth sample preparation part)
C24 RET/PLT-F reaction chamber (fourth sample preparation part)
SEQ1 operation sequence (first and second operation sequences)
SEQ2 operation sequence (first and second operation sequences)
SP1 specimen (first specimen)
SP2 specimen (second specimen)

## Claims

1. An analyzer configured to analyze a specimen, comprising:
first and second sample preparation parts configured to prepare a first measurement sample from the specimen by using a first reagent;
a third sample preparation part configured to prepare a second measurement sample from the specimen by using a second reagent;
an optical measurement part configured to measure the first measurement sample to obtain an optical signal corresponding to white blood cells;
an electric measurement part configured to measure the second measurement sample to obtain an electric signal corresponding to red blood cells; and
a controller programmed to analyze the specimen, based on the optical signal and the electric signal, wherein
the controller is further programmed to
control, according to a plurality of measurement orders, preparation in the first and second sample preparation parts and measurement by the optical measurement part such that at least a part of a first period overlaps with at least a part of a second period,
the first period including a preparation of the first measurement sample by the first sample preparation part and a measurement thereof by the optical measurement part, and
the second period including a preparation of the first measurement sample by the second sample preparation part and a measurement thereof by the optical measurement part.

2. The analyzer of claim 1, wherein
the controller is further programmed to control sample preparation in the first and second sample preparation parts such that:
the first measurement sample is prepared from the first specimen in the first sample preparation part, and
the first measurement sample is prepared from a second specimen to be measured in the second sample preparation part next to the first specimen.

3. The analyzer of claim 1 or 2, wherein
the controller is further programmed to control sample preparation in the first and second sample preparation parts such that, for a plurality of different specimens, preparations of first measurement samples in the first and second sample preparation parts are alternately performed.

4. The analyzer of any one of claims 1 to 3, further comprising a fourth sample preparation part configured to prepare a third measurement sample from the specimen by using a third reagent, wherein
the optical measurement part measures the third measurement sample to further obtain an optical signal corresponding to predetermined blood cells.

5. The analyzer of claim 4, wherein
a time for preparing the first measurement sample is longer than a time for preparing the third measurement sample.

6. The analyzer of claim 5, wherein
the number of sample preparation parts for preparing the first measurement sample is larger than the number of sample preparation parts for preparing the third measurement sample.

7. The analyzer of any one of claims 4 to 6, wherein
the controller is further programmed to control sample preparation in either of the first and second sample preparation parts and the fourth sample preparation part such that preparation of the first measurement sample with respect to the specimen is performed before preparation of the third measurement sample with respect to the specimen.

8. The analyzer of any one of claims 4 to 7, wherein
the controller is further programmed to control sample preparation in either of the first and second sample preparation parts and the fourth sample preparation part such that, for each of a plurality of different specimens, the order of preparations of the first and third measurement samples is the same.

9. The analyzer of any one of claims 1 to 8, wherein
the controller is further programmed to control sample preparation in either of the first and second sample preparation parts and the third sample preparation part such that preparation of the second measurement sample with respect to the specimen is performed before preparation of the first measurement sample with respect to the specimen.

10. The analyzer of any one of claims 1 to 9, wherein
the analysis part generates an analysis result including a measured value regarding a volume of red blood cells, based on the electric signal.

11. The analyzer of any one of claims 1 to 10, wherein
the analysis part generates an analysis result of the specimen such that a sample preparation part used for preparing the first measurement sample becomes identifiable.

12. The analyzer of any one of claims 1 to 11, wherein
the controller is further programmed to control sample preparation in the first and second sample preparation parts such that the first measurement samples are prepared with common preparation conditions.

13. The analyzer of any one of claims 1 to 12, wherein
the first reagent is supplied to the first and second sample preparation parts from a common reagent container, and
the third reagent is supplied to the fourth sample preparation part from a reagent container different from the reagent container that contains the first reagent.

14. The analyzer of any one of claims 1 to 13, further comprising a dispenser configured to dispense the specimen, wherein
the controller is further programmed to control the dispenser such that the same amount of specimen is dispensed to the first and second sample preparation parts.

15. The analyzer of claim any one of claims 1 to 14, wherein
a reaction time of the specimen with the first reagent is common between the first and second sample preparation parts.

16. The analyzer of claim any one of claims 1 to 15, wherein
the amount of the first measurement sample to be prepared is common between the first and second sample preparation parts.

17. The analyzer of any one of claims 1 to 16, wherein
a reaction temperature of the first measurement sample to be prepared is common between the first and second sample preparation parts.

18. The analyzer of any one of claims 1 to 17, wherein
the controller sets a start timing of a second operation sequence corresponding to a second measurement order in which the second sample preparation part is used, according to a first operation sequence corresponding to a first measurement order in which the first sample preparation part is used.

19. The analyzer of any one of claims 1 to 18, wherein
the optical measurement part is shared by measurement of a measurement sample prepared by the first sample preparation part, and measurement of a measurement sample prepared by the second sample preparation part.

20. The analyzer of claim 19, wherein
when performing a first operation sequence corresponding to a first measurement order in which the first sample preparation part is used and a second operation sequence corresponding to a second measurement order in which the second sample preparation part is used, in this order, the controller sets, as a start timing of the second operation sequence, a start timing which is after start and before end of the first operation sequence and which causes measurement of the measurement sample corresponding to the second operation sequence by the optical measurement part to be performed after measurement of the measurement sample corresponding to the first operation sequence by the optical measurement part.
